(19) **Europäisches Patentamt / European Patent Office / Office européen des brevets**

(11) **EP 4 717 267 A1**

(12) **EUROPEAN PATENT APPLICATION**
published in accordance with Art. 153(4) EPC

(43) Date of publication:
**01.04.2026 Bulletin 2026/14**

(21) Application number: **24796361.4**

(22) Date of filing: **23.04.2024**

(51) International Patent Classification (IPC):
*A61K 31/704* (2006.01) *A61K 31/64* (2006.01)
*A61P 35/00* (2006.01) *A61P 35/04* (2006.01)

(52) Cooperative Patent Classification (CPC):
**A61K 31/64; A61K 31/704; A61P 35/00; A61P 35/04**

(86) International application number:
**PCT/ES2024/070255**

(87) International publication number:
**WO 2024/223971 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.04.2023 ES 202330324**

(71) Applicants:
• **FUNDACION UNIVERSITARIA SAN ANTONIO (UCAM)**
  **30107 Guadalupe (Murcia) (ES)**
• **Silesian University of Technology (SUT)**
  **44-100 Gliwice (PL)**
• **Universidad de Lodz**
  **20-013 Lodz (PL)**

(72) Inventors:
• **PÉREZ SÁNCHEZ, Horacio Emilio**
  **30100 MURCIA (ES)**
• **TOMCZYK, Mateusz**
  **44-100 GLIWICE (PL)**
• **LEGIERSKI, Hubert**
  **43-474 KONIAKOW (PL)**
• **MATCZAK, Karolina**
  **90-222 LODZ (PL)**
• **KUBICKA, Anna**
  **95-011 BRATOSZEWICE (PL)**
• **LABIENIEC-WATALA, Magdalena**
  **95-200 PABIANICE (PL)**

(74) Representative: **Díaz Pacheco, Maria Desamparados**
**MIO patentes & marcas**
**Avda. de Granada, S/N**
**Centro de Negocios Vega Plaza - Oficina 14**
**30500 Molina de Segura (Murcia) (ES)**

(54) **SYNERGISTIC COMBINATION FOR TREATING CANCER**

(57) Synergistic drug combinations for anticancer therapy, comprising a therapeutically effective amounts of an anticancer agent and Glimepiride and/or Glimepiride analogs, and a pharmaceutical composition comprising said combination.

EP 4 717 267 A1

**Description**

**FIELD OF THE INVENTION**

The present invention relates to synergistic combination therapies comprising sulfonylurea-based type 2 diabetes drugs, primarily Glimepiride, and an anticancer agent for the treatment of cancer, or a Glimepiride analog and an anticancer agent for the treatment of cancer.

**BACKGROUND OF THE INVENTION**

Cancer is a leading cause of death worldwide, accounting for nearly 10 million deaths in 2020. The most common in 2020 (in terms of new cases of cancer) were: breast (2.26 million cases); lung (2.21 million cases); colon and rectum (1.93 million cases); prostate (1.41 million cases); skin (non-melanoma) (1.20 million cases); and stomach cancers (1.09 million cases). Among the most common causes of cancer death in 2020 were: lung (1.80 million deaths); colon and rectum (916 000 deaths); liver (830 000 deaths); stomach (769 000 deaths); and breast cancers (685 000 deaths).

Despite a tremendous amount of worldwide research and efforts to stem the tide of cancer and its side effects, the disease in its many manifestations continues to be a huge problem. Therefore, any new cancer treatment having a curative affect and/or the ability to ameliorate cancer symptoms and improve the lifestyle of patients is highly significant and important.

The most common treatments for cancer are surgery, chemotherapy, and radiation therapy. All of these techniques have significant drawbacks in terms of side effects and patient discomfort. For example, chemotherapy may result in significant decreases in white blood cell count (neutropenia), red blood cell count (anemia), and platelet count (thrombocytopenia). This can result in pain, diarrhea, constipation, mouth sores, hair loss, nausea, and vomiting. In some cases, surgery is the first and most common treatment, such as in most people with brain cancers. For some, surgical removal may be the only treatment needed. Sometimes, however, the cancer cannot be safely removed because it is too close to certain parts of the brain and surgery would cause blindness, speech problems or partial paralysis. In such cases, radiotherapy or chemotherapy is often used to destroy tumor tissue that cannot be removed, or to kill cancer cells that may remain after surgery.

Breast cancer is the most commonly diagnosed cancer in women worldwide. Currently, there is no widely available effective method to prevent or treat breast cancer. Treatment of the disease currently involves a combination of early diagnosis (through routine breast screening) and aggressive treatment, which may include one or more methods of surgery, radiation therapy, chemotherapy, and hormone therapy. Surgery is often followed by chemotherapy, radiation therapy or hormone blocking therapy, especially if the disease has given metastasis. Although patient survival has improved over the past two decades, metastatic breast cancer is still incurable with the currently available treatment. Breast cancer can give metastases to lymph nodes, skin, lungs, liver, brain, or bone. Metastasis can occur early or late in the disease, although usually metastasis occurs when tumor growth reaches about 20 mm in size. The five-year survival rate for stage 4 breast cancer is 22%; median survival is three years. Annually, the disease takes 40 000 lives. Currently, there are no effective long-term treatments for breast cancer that has metastasized to lymph nodes or distant sites, and the chemotherapy used has many side effects that are unfavorable for patients.

In patients with metastatic breast cancer, chemotherapy regimens containing anthracyclines are one of the first-line treatments, which are routinely used as standard adjuvant or neoadjuvant treatment for high-risk early breast cancer. Doxorubicin belongs to the anthracycline family and is currently the most effective chemotherapeutic drug used to treat breast cancer. However, Doxorubicin has been shown to induce cardiotoxicity, drug resistance, and even tumor growth, resulting in poor prognosis and worse patient survival, so these drugs are generally not considered in patients with metastatic breast cancer. The biggest problem is resistance to chemotherapeutic drugs, which can cause treatment failure in more than 90% of patients with metastatic cancer. Consequently, there is no standard treatment for metastatic breast cancer patients whose disease progressed prior to anthracycline-based chemotherapy to date, and it remains a great challenge to select an appropriate regimen in this setting. Because of that, there is growing interest in developing combination therapies in metastatic breast cancer to overcome these drawbacks, minimize the side effects of anthracycline drugs, give patients more time before tumor dissemination occurs, and prolong overall survival.

Lung cancer is the second-most commonly diagnosed cancer in both men and women, representing about 14% of all newly diagnosed cancers, the most frequent subtype being non-small-cell lung cancer (90%). Unfortunately, more than 70% of lung cancer patients have already developed locally advanced or metastatic disease by the time they are diagnosed, and less than 16% have a 5-year survival rate. Although in these advanced stages, first-line chemotherapy treatment involves Cisplatin and Carboplatin, other associated chemotherapy drugs (e.g., Paclitaxel, Docetaxel, Irinotecan, Topotecan, and Gemcitabine) are often used, with Doxorubicin being among the most effective. Despite good patient-survival rates from these combination-chemotherapy treatments, drug resistance and drug-related toxicity severely limit clinical outcomes. Therefore, strategies that decrease both drug toxicity and resistance may significantly improve lung cancer response to current drug associations.

Chemotherapy for the treatment of brain tumor is often used after surgery for some types of brain tumor, such as gliomas, at the same time as and for several months after radiation therapy, or for brain tumor that has returned after treatment. However, it can be difficult to treat brain tumors with some chemotherapy drugs because the brain is protected by the blood-brain barrier. Medical advances have made it possible to locally deliver chemotherapeutic drugs directly to brain tumors bypassing the blood-brain barrier, which allows high drug concentrations to be achieved at the site of administration while eliminating the toxicity associated with traditional systemic administration of chemotherapeutic drugs. Although various controlled-release therapy strategies are currently being investigated to improve the efficacy of chemotherapy against brain tumors, another potential strategy is to test drugs other than Carmustine, which is the treatment of choice for patients with certain brain cancers. The best candidates for controlled-release therapy against brain tumors are drugs that do not cross the blood-brain barrier, have significant systemic toxicity and are selectively cytotoxic to tumor cells. One such drug is Doxorubicin, which is currently used to treat a wide variety of cancers.

Despite the clinical effectiveness of Doxorubicin in the treatment of many malignant tumors, clinical trials involving systemic administration of the drug have shown very limited efficacy in the treatment of certain types of brain tumors, such as gliomas. Very high doses of Doxorubicin must be administered systemically to achieve any therapeutic benefit, and these doses are highly neurotoxic and, therefore, ineffective in treating central nervous system tumors. The limited efficacy of Doxorubicin when administered systemically can be explained by the poor penetration of the drug through the blood-brain barrier and the glycoprotein-mediated effect of drug efflux from the cerebrospinal fluid

Significantly better results have been obtained with intracerebral administration of Doxorubicin. In one clinical trial, ten patients with stage III or IV gliomas were treated with direct infusion of Doxorubicin through the Ommaya reservoir (S. Voulgaris et al., Am. J. Clin. Oncol. 25 (2002) 60-64).

The combination of Doxorubicin with Glibenclamide, a second-generation oral antidiabetic drug with a sulfonylurea structure, was shown to be effective in inhibiting the proliferation of MDA-MB-231 breast carcinoma cells (M. Núñez et al., BMC Pharmacol. Toxicol. 14 (2013) 6). Administration of Glibenclamide alone produces a cytostatic effect in MDA-MB-231 cells inhibiting the G1-S phase progression without inducing cell death or differentiation. Despite the lack of cytotoxic effect of Glibenclamide, the combination of 25 $\mu$M Glibenclamide plus Doxorubicin in doses over 0.05 nM was found to inhibit cell proliferation more effective than single treatments. For example, while administration of 0.05 nM and 0.1 nM Doxorubicin alone reduces MDA-MB-231 cell viability to 54% of and 48% of untreated control, respectively, administration of Doxorubicin with 25 $\mu$M Glibenclamide reduce cell viability to 33% and 30%, respectively. Although improvements in the cytotoxic activity of the two-drug combination were demonstrated, this was only for the combination of two doses of Doxorubicin (0.05 nM and 0.1 nM) and one dose of Glibenclamide (25 $\mu$M) against one breast cancer cell line (MDA-MB-231), and the authors did not determine whether the cytotoxic activity of the combination behaved additively or synergistically.

## DESCRIPTION OF THE INVENTION

In the present specification the terms "cancer" and "tumor" are synonyms and can be used interchangeably.

In the present specification, the term "synergy" means that the combination of Glimepiride and an anticancer agent or Glimepiride analog and an anticancer agent has a therapeutic effect on cancerous tissue which is greater than the sum of the individual therapeutic effects of Glimepiride and an anticancer agent on the cancerous tissue separately.

Unless otherwise defined, all terms, designations and other scientific terms or terminology used herein have meanings commonly understood by persons skilled in the art to which the present invention relates. In some cases, terms with commonly understood meanings are defined herein for clarity and/or for easy reference, and the inclusion of such definitions herein need not be construed as a significant difference from what is commonly understood in the art. Many of the techniques and procedures described or referred to herein are well understood and commonly used using conventional methodology by those skilled in the art. All publications, patents and patent applications cited herein are hereby incorporated by reference in their entirety for all purposes. In describing a preferred embodiment, reference may be made to the accompanying drawings, which form part of the present document and in which a specific embodiment in which the invention may be used is illustratively shown. It should be understood that other implementations can be used, and structural changes can be made without departing from the scope of the present invention.

A first object of the invention relates to a synergistic composition comprising a combination of

- Glimepiride or
- a Glimepiride analog selected from the group consisting of Tolazamide, Gliclazide, Chlorpropamide, Tolbutamide and Glipizide, and
- an anticancer agent selected from the group consisting of: Doxorubicin, Docetaxel and Capecitabine.

for use in the treatment of cancer

The above mentioned compounds also comprise pharmaceutically acceptable salts, solvates, or stereoisomers thereof.

The IUPAC name of Glimepiride is 4-ethyl-3-methyl-N-[2-[4-[(4-methylcyclohexyl) carbamoylsulfamoyl]phenyl] ethyl]-5-oxo-2H-pyrrole-1-carboxamide

The IUPAC name of Tolazamide is 1-(azepan-1-yl)-3-(4-methylphenyl)sulfonylurea

The IUPAC name of Gliclazide is 1-(3,3a,4,5,6,6a-hexahydro-1H-cyclopenta[c]pyrrol-2-yl)-3-(4-methylphenyl) sulfonylurea

The IUPAC name of Chlorpropamide is 1-(4-chlorophenyl)sulfonyl-3-propylurea

The IUPAC name of Tolbutamide is 1-butyl-3-(4-methylphenyl)sulfonylurea

The IUPAC name of Glipizide is N-[2-[4-(cyclohexylcarbamoylsulfamoyl)phenyl]ethyl]-5-methylpyrazine-2-carboxamide

The IUPAC name of Doxorubicin is (7S,9S)-7-[(2R,4S,5S,6S)-4-amino-5-hydroxy-6 methyloxan-2-yl] oxy-6,9,11-trihydroxy-9-(2-hydroxyacetyl)-4-methoxy-8,10-dihydro-7H tetracene-5,12-dione

The IUPAC name of Capecitabine is pentyl N-[1-[(2R,3R,4S,5R)-3,4-dihydroxy-5-methyloxolan-2-yl]-5-fluoro-2-oxopyrimidin-4-yl]carbamate

The IUPAC name of Docetaxel is [(1S,2S,3R,4S,7R,9S,10S,12R,15S)-4-acetyloxy-1,9,12 trihydroxy-15-[(2R,3S)-2-hydroxy-3-[(2-methylpropan-2-yl)oxycarbonylamino]-3 phenylpropanoyl]oxy-10,14,17,17-tetramethyl-11-oxo-6 oxatetracyclo[11.3.1.03,1 0.04, 7]heptadec-13-en-2-yl] benzoate

In a particular embodiment, the anticancer agent is Doxorubicin

In another particular embodiment the synergistic composition comprises Docetaxel and Glimepiride.

In another particular embodiment the synergistic composition comprises Capecitabine and Glimepiride.

In a particular embodiment, the composition comprises a combination of Doxorubicin and Glimepiride or Doxorubicin and a Glimepiride analog selected from the group consisting of Tolazamide, Gliclazide, Chlorpropamide, Tolbutamide and Glipizide.

Glibenclamide and Gliquidone which are considered analogs of Glimepiride are not under the scope of the present application.

In a particular embodiment the composition comprises a combination of Doxorubicin and Tolazamide, or Doxorubicin and Gliclazide, or Doxorubicin and Chlorpropamide, or Doxorubicin and Tolbutamide, or Doxorubicin and Glipizide

In another particular embodiment the composition comprises a combination of Docetaxel and Tolazamide, or Docetaxel and Gliclazide, or Docetaxel and Chlorpropamide, or Docetaxel and Tolbutamide, or Docetaxel and Glipizide

In another particular embodiment the composition comprises a combination of Doxorubicin and Tolazamide, or Doxorubicin and Gliclazide, or Doxorubicin and Chlorpropamide, or Doxorubicin and Tolbutamide, or Doxorubicin and Glipizide

In another particular embodiment the composition comprises a combination of Capecitabine and Tolazamide, or Capecitabine and Gliclazide, or Capecitabine and Chlorpropamide, or Capecitabine and Tolbutamide, or Capecitabine and Glipizide

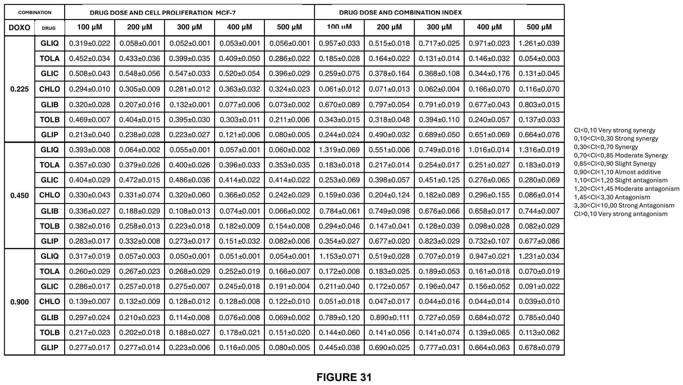

In the case of Gliquidone, it will be shown in the next section that not every combination of concentrations of Doxorubicin and Gliquidone produce a synergistic effect (FIG. 31), thus a skilled person, in the light of the present disclosure and applying the common general knowledge would know which concentrations of the compounds are suitable to get a synergistic effect for the treatment of cancer.

The inventors of the present invention have established that Glimepiride administered in combination with an anticancer agent selected from the group consisting of: Doxorubicin, Docetaxel and Capecitabine, preferably Doxorubicin, or a Glimepiride analog administered in combination with an anticancer agent selected from the group consisting of: Doxorubicin, Docetaxel and Capecitabine, preferably Doxorubicin, has additive or synergistic effects and is capable of significantly reducing the hyperproliferative activity of human cancer cells. It is anticipated that said anticancer agents used in combination with Glimepiride or a Glimepiride analog, can give significantly enhanced cytotoxic or antitumor effects on cancer cells *in vivo,* thus providing an enhanced therapeutic effect. In particular, a significantly enhanced growth inhibitory effect is obtained with the above disclosed combination using lower concentrations of anticancer drug compared to treatment regimens in which the anticancer agent selected from the group consisting of: Doxorubicin, Docetaxel and Capecitabine is used alone. This combination provides a therapy in which the side effects associated with Doxorubicin used alone at higher doses are absent or significantly reduced. Alternatively, this combination therapy makes it possible to obtain a greater antiproliferative effect from the usual dose of anticancer drugs used in standard treatment regimens, increasing the effectiveness of therapy and/or reducing the total number of treatments required.

Another advantage of the invention is that the synergistic combination allows for a reduce concentration of Glimepiride or a Glimepiride analog, and/or the anticancer agent selected from the group consisting of: Doxorubicin, Docetaxel and Capecitabine which leads to a reduction of the treatment time and/or accompanying side effects.

As used herein, the term "a combination of," when referring to two or more compounds, agents, or additional active pharmaceutical ingredients, means the administration of two or more compounds within a composition, agents, or active pharmaceutical ingredients to the subject prior to, concurrent with, or subsequent to each other.

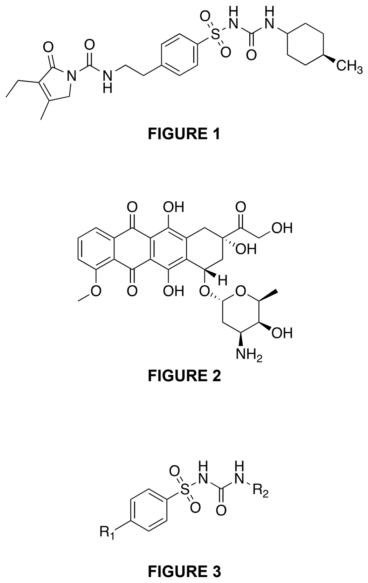

Glimepiride and its abbreviation GLIM refers to a compound having a structure represented by the formula shown in FIG. 1. GLIM also comprises any of its pharmaceutically acceptable salts, esters, tautomers, isomers, pharmaceutically acceptable solvates or hydrates, or any combination thereof.

Tolazamide and its abbreviation TOLA, Gliclazide and its abbreviation GLIC, Chlorpropamide and its abbreviation CHLO, Tolbutamide and its abbreviation TOLB, Glipizide and its abbreviation GLIP also comprise any of their pharmaceutically acceptable salts, esters, tautomers, isomers, pharmaceutically acceptable solvates or hydrates, or any combination thereof.

Doxorubicin and its abbreviation DOX refers to a compound having a structure represented by the formula shown in FIG. **2.** Doxorubicin is a member of the anthracycline antibiotic family of chemotherapeutics. It acts by intercalating into DNA, so that DNA processing cannot occur. The tumor cells cannot divide, and eventually they will die. DOX also comprises any of their pharmaceutically acceptable salts, esters, tautomers, isomers, pharmaceutically acceptable solvates or hydrates, or any combination thereof.

Docetaxel and Capecitabine and their abbreviations DOCE and CAPE. DOCE belongs to the taxane family of compounds and acts by stopping the growth of cancer cells. CAPE is a prodrug which inside the body, is converted to 5-fluorouracil (5-FU) through which it acts. It belongs to the class of medications known as fluoropyrimidines, DOCE and CAPE also comprises any of their pharmaceutically acceptable salts, esters, tautomers, isomers, pharmaceutically acceptable solvates or hydrates, or any combination thereof.

In a particular embodiment the composition may have one or more further, Glimiperide and/or Glimepiride analogs selected from: Tolazamide, Gliclazide, Chlorpropamide, Tolbutamide and Glipizide.

For example: Doxorubicin, Glimepiride and Tolazamide, or Doxorubicin, Glimepiride and Gliclazide, or Doxorubicin, Glimepiride and Chlorpropamide, or Doxorubicin, Glimepiride and Tolbutamide, or Doxorubicin, Glimepiride and Glipizide, or Doxorubicin, Tolazamide and Gliclazide, or Doxorubicin, Tolazamide and Chlorpropamide, or Doxorubicin, Tolazamide and Tolbutamide, or Doxorubicin, Tolazamide and Glipizide, or Doxorubicin, Gliclazide and Chlorpropamide, or Doxorubicin, Gliclazide and Tolbutamide, or Doxorubicin, Gliclazide and Glipizide, or Doxorubicin, Chlorpropamide and Tolbutamide, or Doxorubicin, Chlorpropamide and Glipizide, or Doxorubicin, Tolbutamide and Glipizide.

In another particular embodiment: Docetaxel, Glimepiride and Tolazamide, or Docetaxel, Glimepiride and Gliclazide, or Docetaxel, Glimepiride and Chlorpropamide, or Docetaxel, Glimepiride and Tolbutamide, or Docetaxel, Glimepiride and Glipizide, or Docetaxel, Tolazamide and Gliclazide, or Docetaxel, Tolazamide and Chlorpropamide, or Docetaxel, Tolazamide and Tolbutamide, or Docetaxel, Tolazamide and Glipizide, or Docetaxel, Gliclazide and Chlorpropamide, or Docetaxel, Gliclazide and Tolbutamide, or Docetaxel, Gliclazide and Glipizide, or Docetaxel, Chlorpropamide and Tolbutamide, or Docetaxel, Chlorpropamide and Glipizide, or Docetaxel, Tolbutamide and Glipizide.

In another particular embodiment: Capecitabine, Glimepiride and Tolazamide, or Capecitabine, Glimepiride and Gliclazide, or Capecitabine, Glimepiride and Chlorpropamide, or Capecitabine, Glimepiride and Tolbutamide, or Capecitabine, Glimepiride and Glipizide, or Capecitabine, Tolazamide and Gliclazide, or Capecitabine, Tolazamide and Chlorpropamide, or Capecitabine, Tolazamide and Tolbutamide, or Capecitabine, Tolazamide and Glipizide, or Capecitabine, Gliclazide and Chlorpropamide, or Capecitabine, Gliclazide and Tolbutamide, or Capecitabine, Gliclazide and Glipizide, or Capecitabine, Chlorpropamide and Tolbutamide, or Capecitabine, Chlorpropamide and Glipizide, or Capecitabine, Tolbutamide and Glipizide.

Specifically, the methods of the present invention are useful for treating and/or preventing cancer and/or inhibiting cancer cell proliferation.

By "pharmaceutically acceptable" is meant a material that is not biologically or otherwise undesirable, i.e., the material can be administered to a subject along with the selected compound without causing any undesirable biological effects or interacting in a deleterious manner with any of the other components of the pharmaceutical composition in which it is contained.

The term "preventing" as used herein refers to administering the combination of the invention prior to the onset of clinical symptoms of a disease or conditions so as to prevent a physical manifestation of aberrations associated with the disease or condition. In the context of cancer, the term "preventing" refers to administering a compound prior to the onset of clinical symptoms of cancer so as to prevent a physical manifestation of aberrations associated with cancer.

The terms "treatment" and "treating" as used herein refer to the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, amelioration, stabilization or prevention. The effects of treatment can be measured or assessed as described herein and as known in the art as is suitable for the disease, pathological condition, or disorder involved. Such measurements and assessments can be made in qualitative and/or quantitative terms. Thus, for example, characteristics or features of a disease, pathological condition, or disorder and/or symptoms of a disease, pathological condition, or disorder can be reduced to any effect or to any amount. In the context of a subject suffering from cancer, the terms "treatment" and "treating" refer to the medical management of a subject with the intent to cure, ameliorate, or stabilize cancer. In the context of a subject at risk of developing cancer, the terms "treatment" and "treating" refer to the medical management of a subject with the intent to prevent cancer.

While Glimepiride and Doxorubicin are main illustrative working embodiments in this disclosure, it is to be noted that Glimepiride and Doxorubicin may be substituted with their analogs and derivatives, in one or more embodiments of the invention.

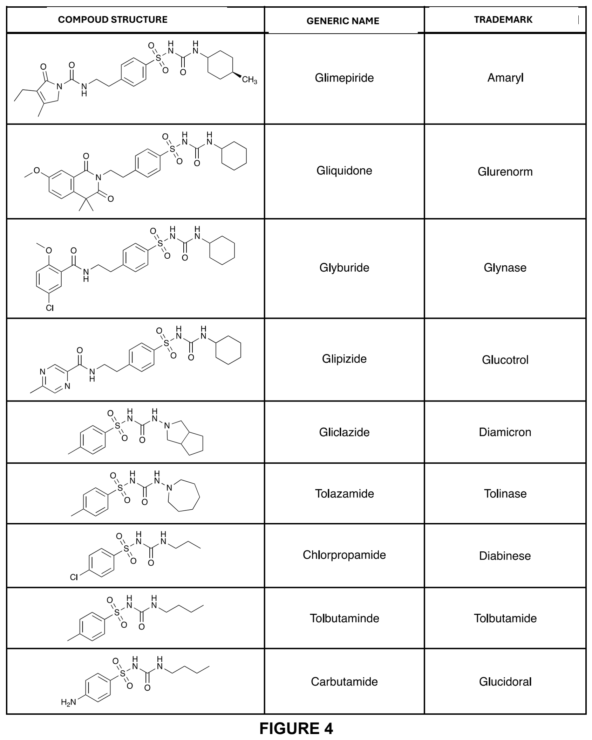

Glimepiride belongs to a class of sulfonylurea drugs whose common chemical core structure is shown in FIG. 3, where $R_1$ and $R_2$ denote chemical groups or atoms. Glimepiride and its analogs are drugs used in the management and treatment of type 2 diabetes. FIG. 4 shows the chemical structures of the sulfonylurea-based Glimepiride family, which are FDA-approved drugs used to treat type 2 diabetes.

In a particular embodiment, the combination of the present invention is useful to reduce the side effects of the anticancer agent selected from the group consisting of: Doxorubicin, Docetaxel and Capecitabine. In the particular case of Doxorubicin the side effects associated with said drug treatment are:

Hematologic side effects: leukopenia, neutropenia, anemia, thrombocytopenia, hemorrhage

Gastrointestinal side effects: anorexia, nausea/vomiting, dehydration, mucositis/stomatitis, hyperpigmentation of the oral mucosa, esophagitis, abdominal pain, gastric erosions, gastrointestinal tract bleeding, diarrhea, colitis

Hepatic side effects: changes in transaminase levels, hyperuricemia

Endocrine side effects: amenorrhea, hot flashes, oligospermia, azoospermia, weight gain

Ocular side effects: conjunctivitis/keratitis, lacrimation

Cutaneous side effects: alopecia, local toxicity, rash/itch, skin changes, severe local tissue necrosis with intravenous injection, extravasation may occur, skin and nail hyperpigmentation, photosensitivity, hypersensitivity to irradiated skin ('radiation recall reaction'), urticaria, acral erythema, palmar plantar erythrodysesthesia

Vascular side effects: phlebitis, thrombophlebitis, thromboembolism

Urological side effects: red coloration of urine for 1 to 2 days after administration

Bladder side effects: pain, hemorrhage, and occasionally decreased bladder capacity upon instillation

Local side effects: severe cellulitis, vesication, tissue necrosis upon extravasation, erythematous streaking along the vein proximal to the site of the injection

Other side effects: anaphylaxis, infection, sepsis/septicemia, acute lymphocytic leukemia, acute myelogenous leukemia, malaise/asthenia, fever, chills, shock, cross sensitivity to lincomycin

In another particular embodiment, the combination of the invention lead to a reduction in the risk of secondary neoplasms. Particularly those that are in a latent stage such as secondary leukemia. Secondary leukemia, with or without a preleukemic phase, has been reported in patients treated with topoisomerase-II inhibitors including the anthracyclines such as doxorubicin. Secondary leukemia is more common when anthracyclines are given in combination with DNA-damaging antineoplastic agents (0.5%) and/or in combination with radiotherapy (2.5 %) with a risk estimated at 1.5% at 10 years. Secondary leukemia can have a 1-3 year latency period, and can occur as late as 10 years following treatment. Therefore, the combination of the invention can prevent the onset of further cancers.

In another particular embodiment, the invention relates to a combination of Glimepiride or Glimperide analogs and at least one anticancer agent for use in the treatment of cancer, wherein:

(a)Glimepiride or a Glimperide analog is administered to a patient to establish a therapeutically effective plasma level for a period of at least one week, and

(b)At least one anticancer agent selected from the group consisting of Doxorubicin, Docetaxel and Capecitabine is administered to establish a period with a therapeutically effective plasma level, and

(c)said periods of a) and b) overlap.

In a preferred embodiment, Doxorubicin is the preferred anticancer agent

Alternatively, the subject may already be undergoing chemotherapy treatment with the anticancer agent selected from the group consisting of: Doxorubicin, Docetaxel and Capecitabine, in which case, Glimepiride or a Glimperide analog is added as an adjunctive therapy to the anticancer agent (and vice versa). Administration of the anticancer agent may occur prior to, simultaneously, or after administration of Glimepiride or a Glimperide analog. All routes of administration of Glimepiride or a Glimperide analog or its simultaneous administration with the anticancer agent are suitable.

In a particular embodiment, a subject is administered with Glimepiride or a Glimperide analog until a suitable concentration is reached, and then, said subject is treated with the anticancer agent. A skilled person would know that as these components are accumulated at a different speed in the human body, so he or she would know using common general knowledge which compound has to be administered first in order to reach a suitable concentration and then adminsitere the other compound, the one that is absorbed faster so the synergy concentration of the combination is reached.

As used herein, "subject" includes, animals and humans. The subject can be a vertebrate, more specifically a mammal (e.g., a human, horse, pig, rabbit, dog, sheep, goat, non-human primate, cow, cat, guinea pig or rodent), a fish, a bird or a reptile or an amphibian. The term does not denote a particular age or sex. Thus, adult and newborn subjects, as well as fetuses, whether male or female, are intended to be covered. A patient refers to a subject afflicted with a disease or disorder. The term "patient" includes human and veterinary subjects. In one aspect, the compounds described herein can be administered to a subject comprising a human or an animal including, but not limited to, a mouse, dog, cat, horse, bovine or ovine and the like, that is in need of alleviation or amelioration from a recognized medical condition. In the present specification "subject" and "patient" are synonyms and can be used interchangeably

By the term "effective amount" of a compound as provided herein is meant a nontoxic but sufficient amount of the compound to provide the desired result, without excluding that to achieve the desired results, the subject may experience side effects. As will be pointed out below, the exact amount required will vary from subject to subject, depending on the species, age, and general condition of the subject, the severity of the disease that is being treated, the particular compound used, its mode of administration, and the like. Thus, it is not possible to specify an exact "effective amount." However, an appropriate effective amount can be determined by one of ordinary skill in the art using only routine experimentation. In the context of compounds and compositions for prevention cancer, the term "effective amount" of a compound as provided herein is meant a nontoxic but sufficient amount of the compound to prevent cancer. In the context of compounds and compositions for treatment of a subject suffering from cancer, the term "effective amount" of a compound as provided herein is meant a nontoxic but sufficient amount of the compound to treat cancer.

In a particular embodiment of the invention the cancer is selected from breast cancer, e.g. ductal carcinoma in situ (DCIS), invasive ductal carcinoma (IDC), invasive lobular carcinoma (ILC), inflammatory breast cancer, triple-negative breast cancer, HER2-positive breast cancer; lung cancer, e.g. non-small cell lung cancer (NSCLC), small cell lung cancer (SCLC), adenocarcinoma, squamous cell carcinoma, large cell carcinoma, carcinoid tumor; brain cancer, e.g. glioblastoma multiforme, astrocytoma, oligodendroglioma, ependymoma, medulloblastoma, meningioma; lymphoblastic leukemia, e.g. acute lymphoblastic leukemia (ALL), B-cell lymphoblastic leukemia, T-cell lymphoblastic leukemia, philadelphia chromosome-positive (Ph+) ALL, hypodiploid ALL, early T-cell precursor (ETP) ALL; acute myeloblastic leukemia, e.g. acute promyelocytic leukemia (APL), acute myelomonocytic leukemia (AMML), acute megakaryoblastic leukemia (AMKL), acute erythroid leukemia (AEL), acute monocytic leukemia (AML-M5), acute basophilic leukemia (ABL); Hodgkin lymphoma, e.g. nodular sclerosis Hodgkin lymphoma (NSHL), mixed cellularity Hodgkin lymphoma (MCHL), lymphocyte-rich Hodgkin lymphoma (LRHL), lymphocyte-depleted Hodgkin lymphoma (LDHL), nodular lymphocyte-predominant Hodgkin lymphoma (NLPHL); non-Hodgkin lymphoma (NHL), e.g. diffuse large B-cell lymphoma (DLBCL), follicular lymphoma (FL), mantle cell lymphoma (MCL), marginal zone lymphoma (MZL), burkitt lymphoma (BL), waldenstrom macroglobulinemia (WM), T-cell lymphomas (TCL); metastatic breast cancer, e.g. invasive ductal carcinoma (IDC), invasive lobular carcinoma (ILC), triple-negative breast cancer (TNBC), HER2-positive breast cancer, inflammatory breast cancer, male breast cancer; metastatic Wilms' tumor; metastatic neuroblastoma; metastatic soft tissue sarcoma, e.g. liposarcoma, leiomyosarcoma, synovial sarcoma, gastrointestinal stromal tumor (GIST), rhabdomyosarcoma, Ewing sarcoma; metastatic bone sarcoma, e.g. osteosarcoma, chondrosarcoma, Ewing sarcoma, giant cell tumor of bone, adamantinoma, fibrosarcoma; metastatic ovarian carcinoma, e.g. high-grade serous carcinoma, endometrioid carcinoma; clear cell carcinoma, mucinous carcinoma, brenner tumor; metastatic transitional cell, e.g. urothelial carcinoma of the bladder, ureteral carcinoma; renal pelvis carcinoma, squamous cell carcinoma of the bladder (which is a rare subtype of TCC); bladder carcinoma, e.g. urothelial carcinoma (also known as transitional cell carcinoma), squamous cell carcinoma, adenocarcinoma, small cell carcinoma (which is a rare subtype of bladder cancer); metastatic thyroid carcinoma, e.g. papillary thyroid carcinoma, follicular thyroid carcinoma, medullary thyroid carcinoma, anaplastic thyroid carcinoma; metastatic gastric carcinoma, e.g. adenocarcinoma, signet ring cell carcinoma, lymphoma, gastrointestinal stromal tumor (GIST), carcinoid tumor; metastatic bronchogenic carcinoma, e.g. small cell lung cancer (SCLC), non-small cell lung cancer (NSCLC); or combinations thereof, preferably breast cancer, lung cancer and brain cancer.

In another particular embodiment the cancer is a primary tumor or a metastatic cancer.

The combination of the invention comprises a therapeutically effective amount of Glimepiride or a Glimperide analog and a therapeutically effective amount of the anticancer agent. As used herein, a "therapeutically effective" amount refers to the amount that will elicit the biological or medical response of a tissue, system, or subject that is being sought by a researcher or clinician, and in particular elicit some desired therapeutic effect as against the cancerous tissue by preventing and/or inhibiting proliferation and/or survival of cancerous cells, and/or slowing the progression of the cancer. A skilled person recognizes that an amount may be considered therapeutically "effective" even if the condition is not totally eradicated or prevented, but it or its symptoms and/or effects are improved or alleviated partially in the subject.

The fact that Glimepiride, Glimepiride analogs and the anticancer agent: Doxorubicin, Docetaxel and Capecitabine are well-known drugs that have been used in patients for years to treat cancers and other syndromes/diseases allows information from current and previous studies on the dose and efficacy of these agents to be used to identify doses for the dual drug therapies disclosed herein (e.g., for breast cancer). For Doxorubicin, please see e.g., ADRIAMYCIN PFS® Product Monograph, Pfizer Canada Inc., Submission Control #152892 and Erttmann, R., Erb, N., Steinhoff, A. et al. Pharmacokinetics of doxorubicin in man: dose and schedule dependence. J Cancer Res Clin Oncol 114, 509-513 (1988).

The recommended dosage of Doxorubicin when used as a single agent is 60 mg/m$^2$ to 75 mg/m$^2$ intravenously every 21 days. An alternative dose schedule is weekly doses of 20 mg/m$^2$, which has been reported to produce a lower incidence of congestive heart failure. A dose of 30 mg/m$^2$ on each of 3 successive days repeated every 4 weeks has also been used. The recommended dosage of Doxorubicin when administered in combination with other chemotherapy drugs, is 40 mg/m$^2$ to 75 mg/m$^2$ intravenously every 21 to 28 days.

In use, a therapeutically-effective amount of the therapeutic combination is administered to a Subject in need thereof. In some embodiments, a composition comprising a therapeutically-effective amount of the therapeutic combination (i.e., as a single unit dosage) is administered to a Subject. In some embodiments, a therapeutically effective amount of Glimepiride and a therapeutically effective amount of Doxorubicin are co-administered to the subject in need thereof. The phrase "co-administer" is intended to embrace administration of each agent in a sequential manner as well as co-administration of these agents in a substantially simultaneous manner in doses given separately. Although described herein with respect to therapeutic treatments, the methods can be also applied for clinical research and/or study.

In a particular embodiment, the amount of Doxorubicin administered to a subject range from 0.001 $\mu$g/ml to 10 $\mu$g/ml. The molecular weight of DOX is 543.52 g/mol, thus a concentration of 0.1 $\mu$M or 1 $\mu$M is equivalent to 0.054 $\mu$g/ml and 0.54 $\mu$g/ml

For example, the concentration of Doxorubicin can be about any one of 0.001 $\mu$g/ml, 0.002 $\mu$g/ml, 0.004 $\mu$g/ml, 0.006 $\mu$g/ml, 0.008 $\mu$g/ml, 0.01 $\mu$g/ml, 0.015 $\mu$g/ml, 0.02 $\mu$g/ml, 0.025 $\mu$g/ml, 0.03 $\mu$g/ml, 0.035 $\mu$g/ml, 0.04 $\mu$g/ml, 0.045 $\mu$g/ml, 0.05 $\mu$g/ml, 0.055 $\mu$g/ml, 0.06 $\mu$g/ml, 0.065 $\mu$g/ml, 0.07 $\mu$g/ml, 0.075 $\mu$g/ml, 0.08 $\mu$g/ml, 0.085 $\mu$g/ml, 0.09 $\mu$g/ml, 0.095 $\mu$g/ml, 0.1 $\mu$g/ml, 0.2$\mu$g/ml, 0.3$\mu$g/ml, 0.4 $\mu$g/ml, 0.5 $\mu$g/ml, 0.6$\mu$g/ml, 0.7$\mu$g/ml, 0.8 $\mu$g/ml, 0.9 $\mu$g/ml, 0.10 $\mu$g/ml, 0.12 $\mu$g/ml, 0.14 $\mu$g/ml, 0.15 $\mu$g/ml, 0.16 $\mu$g/ml, 0.18 $\mu$g/ml, 0.20 $\mu$g/ml, 0.25 $\mu$g/ml, 0.30 $\mu$g/ml, 0.35 $\mu$g/ml, 0.40 $\mu$g/ml, 0.45 $\mu$g/ml, 0.50 $\mu$g/ml, 0.55 $\mu$g/ml, 0.60 $\mu$g/ml, 0.65 $\mu$g/ml, 0.70 $\mu$g/ml, 0.75 $\mu$g/ml, 0.80 $\mu$g/ml, 0.85 $\mu$g/ml, 0.90 $\mu$g/ml, 0.95 $\mu$g/ml, 1 $\mu$g/ml, 1.10 $\mu$g/ml, 1.20 $\mu$g/ml, 1.25 $\mu$g/ml, 1.30 $\mu$g/ml, 1.40 $\mu$g/ml, 1.50 $\mu$g/ml, 1.60 $\mu$g/ml, 1.70 $\mu$g/ml, 1.75 $\mu$g/ml, 1.80 $\mu$g/ml, 1.90 $\mu$g/ml, 2.00 $\mu$g/ml, 2.20 $\mu$g/ml, 2.40 $\mu$g/ml, 2.50 $\mu$g/ml, 2.60 $\mu$g/ml, 2.80 $\mu$g/ml, 3. $\mu$g/ml, 3.20 $\mu$g/ml, 3.40 $\mu$g/ml, 3.50 $\mu$g/ml, 3.60 $\mu$g/ml, 3.80 $\mu$g/ml, 4.0 $\mu$g/ml, 4.20 $\mu$g/ml, 4.40 $\mu$g/ml, 4.50 $\mu$g/ml, 4.60 $\mu$g/ml, 4.80 $\mu$g/ml, or 5.00 $\mu$g/ml, 5.5 $\mu$g/ml, 6.0 pg/ml, 6.5 $\mu$g/ml, 7.0 $\mu$g/ml, 7.5 $\mu$g/ml, 8.0 $\mu$g/ml, 8.5 $\mu$g/ml, 9.0 $\mu$g/ml, 9.5 $\mu$g/ml, 10 $\mu$g/ml.

In a particular embodiment, the amount of Glimepiride administered to a subject range from 1 $\mu$g/ml to 1000 $\mu$g/ml. The molecular weight of Glimepiride is 490.617 g/ml, thus a concentration of 10 $\mu$M or 500 $\mu$M is equivalent to 4,9 $\mu$g/ml and 490,6 $\mu$g/ml

For example, the concentration of Glimepiride can be about any one of 1 $\mu$g/ml, 2$\mu$g/ml, 3$\mu$g/ml, 4 $\mu$g/ml, 5 $\mu$g/ml, 6$\mu$g/ml, 7$\mu$g/ml, 8 $\mu$g/ml, 9 $\mu$g/ml, 10 $\mu$g/ml, 12 $\mu$g/ml, 14 $\mu$g/ml, 15 $\mu$g/ml, 16 $\mu$g/ml, 18 $\mu$g/ml, 20 $\mu$g/ml, 25 $\mu$g/ml, 30 $\mu$g/ml, 35 $\mu$g/ml, 40 $\mu$g/ml, 45 $\mu$g/ml, 50 $\mu$g/ml, 55 $\mu$g/ml, 60 $\mu$g/ml, 65 $\mu$g/ml, 70 $\mu$g/ml, 75 $\mu$g/ml, 80 $\mu$g/ml, 85 $\mu$g/ml, 90 $\mu$g/ml, 95 $\mu$g/ml, 100 $\mu$g/ml, 110 $\mu$g/ml, 120 $\mu$g/ml, 125 $\mu$g/ml, 130 $\mu$g/ml, 140 $\mu$g/ml, 150 $\mu$g/ml, 160 $\mu$g/ml, 170 $\mu$g/ml, 175 $\mu$g/ml, 180 $\mu$g/ml, 190 $\mu$g/ml, 200 $\mu$g/ml, 220 $\mu$g/ml, 240 $\mu$g/ml, 250 $\mu$g/ml, 260 $\mu$g/ml, 280 $\mu$g/ml, 300 $\mu$g/ml.

In a particular embodiment, the combination of Doxorubicin and Glimepiride comprise between about 0.01 $\mu$M to 5 $\mu$M of Doxorubicin and between about 20 $\mu$M to 1000 $\mu$M of Glimepiride, which is equivalent of about 0.006 $\mu$g/ml to 2.75 $\mu$g/ml $\mu$g/ml of Doxorubicin and about 9.81 $\mu$g/ml to 490.6 $\mu$g/ml of Glimepiride.

In a particular embodiment, the combination of Doxorubicin and Glimepiride comprise between about 0.1 $\mu$M to 3 $\mu$M of Doxorubicin and between about 20 $\mu$M to 750 $\mu$M of Glimepiride, which is equivalent of about 0.06 $\mu$g/ml to 1.65 $\mu$g/ml $\mu$g/ml of Doxorubicin and about 9.81 $\mu$g/ml to 367.95 $\mu$g/ml of Glimepiride.

In a particular embodiment, the combination of Doxorubicin and Glimepiride comprise between about 0.01 $\mu$M to 1 $\mu$M of Doxorubicin and between about 20 $\mu$M to 400 $\mu$M of Glimepiride, which is equivalent of about 0.006 $\mu$g/ml to 0.55 $\mu$g/ml of Doxorubicin and about 9.81 $\mu$g/ml to 196.24 $\mu$g/ml of Glimepiride.

In another particular embodiment, the combination of Doxorubicin and Glimepiride comprise between about 0.05 $\mu$M to 0.8 $\mu$M of Doxorubicin and between about 30 $\mu$M to 300 $\mu$M of Glimepiride, which is equivalent of about 0.028 $\mu$g/ml to 0.44 $\mu$g/ml of Doxorubicin and about 14.72 $\mu$g/ml to 147.18 $\mu$g/ml of Glimepiride.

In another particular embodiment, the combination of Doxorubicin and Glimepiride comprise between about 0.1 $\mu$M to 0.5 $\mu$M of Doxorubicin and between about 40 $\mu$M to 200 $\mu$M of Glimepiride, which is equivalent of about 0.055 $\mu$g/ml to 0.275 $\mu$g/ml of Doxorubicin and about 19.62 $\mu$g/ml to 98.12 $\mu$g/ml of Glimepiride. This combination yielded a 4.1-fold higher cancer cell killing efficiency (87% dead cancer cells) than Doxorubicin alone.

In another particular embodiment, the combination of Doxorubicin and Glimepiride comprise between about 0.2 $\mu$M to 0.4 $\mu$M of Doxorubicin and between about 50 $\mu$M to 150 $\mu$M of Glimepiride, which is equivalent of about 0.11 $\mu$g/ml to 0.22 $\mu$g/ml of Doxorubicin and about 24.53 $\mu$g/ml to 73.59 $\mu$g/ml of Glimepiride. This combination yielded a 4.1-fold higher cancer cell killing efficiency (87% dead cancer cells) than Doxorubicin alone.

In another particular embodiment, the combination of Doxorubicin and Glimepiride comprise between about 0.08 $\mu$M to 0.15 $\mu$M of Doxorubicin and between about 20 $\mu$M to 200 $\mu$M of Glimepiride, preferably about 20 $\mu$M to 100 $\mu$M of Glimepiride, which is equivalent of about 0.044 $\mu$g/ml to 0.083 $\mu$g/ml of Doxorubicin and about 9.812 $\mu$g/ml to 98.2 $\mu$g/ml of Glimepiride or 9.81 $\mu$g/ml to 49.1 $\mu$g/ml of Glimepiride. These combinations result in a strong synergistic effect.

In another particular embodiment, the above mentioned concentrations of the combination Doxorubicin and Glimepiride of the preceding seven paragraphs are for use against breast cancer.

In another particular embodiment, the combination of Doxorubicin and Glimepiride comprise between about 0.01 $\mu$M to 1 $\mu$M of Doxorubicin and between about 20 $\mu$M to 1000 $\mu$M of Glimepiride, which is equivalent of about 0.006 $\mu$g/ml to 0.55 $\mu$g/ml of Doxorubicin and about 9.81 $\mu$g/ml to 490.6 $\mu$g/ml of Glimepiride.

In another particular embodiment, the combination of Doxorubicin and Glimepiride comprise between about 0.01 $\mu$M to 1 $\mu$M of Doxorubicin and between about 150 $\mu$M to 1000 $\mu$M of Glimepiride, which is equivalent of about 0.006 $\mu$g/ml to 0.55 $\mu$g/ml of Doxorubicin and about 73.59 $\mu$g/ml to 490.6 $\mu$g/ml of Glimepiride.

In another particular embodiment, the combination of Doxorubicin and Glimepiride comprise between about 0.01 $\mu$M to 1 $\mu$M of Doxorubicin and between about 250 $\mu$M to 1000 $\mu$M of Glimepiride, which is equivalent of about 0.006 $\mu$g/ml to 0.55 $\mu$g/ml of Doxorubicin and about 122.65 $\mu$g/ml to 490.6 $\mu$g/ml of Glimepiride.

In another particular embodiment, the combination of Doxorubicin and Glimepiride comprise between about 0.2 $\mu$M to 1 $\mu$M of Doxorubicin and between about 20 $\mu$M to 1000 $\mu$M of Glimepiride, which is equivalent of about 0.11 $\mu$g/ml to 0.55 $\mu$g/ml of Doxorubicin and about 9.812 $\mu$g/ml to 490.6 $\mu$g/ml of Glimepiride.

In another particular embodiment, the combination of Doxorubicin and Glimepiride comprise between about 0.25 $\mu$M to 0.6 $\mu$M of Doxorubicin and between about 50 $\mu$M to 1000 $\mu$M of Glimepiride, which is equivalent of about 0.138 $\mu$g/ml to 0.33 $\mu$g/ml of Doxorubicin and about 24.53 $\mu$g/ml to 490.6 $\mu$g/ml of Glimepiride. This combination yielded a 1.8-fold increase in anticancer activity.

In another particular embodiment, the combination of Doxorubicin and Glimepiride comprise between about 0.25 $\mu$M to 0.6 $\mu$M of Doxorubicin and between about 50 $\mu$M to 500 $\mu$M of Glimepiride, which is equivalent of about 0.138 $\mu$g/ml to 0.33 $\mu$g/ml of Doxorubicin and about 24.53 $\mu$g/ml to 245.30 $\mu$g/ml of Glimepiride.

In another particular embodiment, the combination of Doxorubicin and Glimepiride comprise between about 0.25 $\mu$M to 0.6 $\mu$M of Doxorubicin and between about 50 $\mu$M to 200 $\mu$M of Glimepiride, which is equivalent of about 0.1375 $\mu$g/ml to 0.33 $\mu$g/ml of Doxorubicin and about 24.53 $\mu$g/ml to 98.12 $\mu$g/ml of Glimepiride. This combination yielded a 1.8-fold increase in anticancer activity

In another particular embodiment, the combination of Doxorubicin and Glimepiride comprise between about 0.3 $\mu$M to 0.9 $\mu$M of Doxorubicin and between about 50 $\mu$M to 175 $\mu$M of Glimepiride, which is equivalent of about 0.17 $\mu$g/ml to 0.5 $\mu$g/ml of Doxorubicin and about 24.53 $\mu$g/ml to 85.86 $\mu$g/ml of Glimepiride. This combination yielded a 2.9-fold increase in anticancer activity.

In another particular embodiment, the eight previous paragraphs relating to the combination of Doxorubicin and Glimepiride are for use against lung cancer.

In another particular embodiment, the combination of Doxorubicin and Glimepiride comprise between about 0.1 $\mu$M to 5 $\mu$M of Doxorubicin and between about 20 $\mu$M to 1000 $\mu$M of Glimepiride, which is equivalent of about 0.055 $\mu$g/ml to 2.75 $\mu$g/ml of Doxorubicin and about 9.812 $\mu$g/ml to 490.6 $\mu$g/ml of Glimepiride.

In another particular embodiment, the combination of Doxorubicin and Glimepiride comprise between about 0.2 $\mu$M to 4 $\mu$M of Doxorubicin and between about 50 $\mu$M to 700 $\mu$M of Glimepiride, which is equivalent of about 0.11 $\mu$g/ml to 2.2 $\mu$g/ml of Doxorubicin and about 24.53 $\mu$g/ml to 343.42 $\mu$g/ml of Glimepiride.

In another particular embodiment, the combination of Doxorubicin and Glimepiride comprise between about 0.2 $\mu$M to 1.5 $\mu$M of Doxorubicin and between about 50 $\mu$M to 350 $\mu$M of Glimepiride, which is equivalent of about 0.11 $\mu$g/ml to 0.84 $\mu$g/ml of Doxorubicin and about 24.53 $\mu$g/ml to 171.71 $\mu$g/ml of Glimepiride.

In another particular embodiment, the combination of Doxorubicin and Glimepiride comprise between about 1.5 $\mu$M to 4 $\mu$M of Doxorubicin and between about 450 $\mu$M to 700 $\mu$M of Glimepiride, which is equivalent of about 0.84 $\mu$g/ml to 2.2 $\mu$g/ml of Doxorubicin and about 220.77 $\mu$g/ml to 343.42 $\mu$g/ml of Glimepiride.

In another particular embodiment, the combination of Doxorubicin and Glimepiride comprise between about 0.1 $\mu$M to 1.5 $\mu$M of Doxorubicin and between about 20 $\mu$M to 250 $\mu$M of Glimepiride, which is equivalent of about 0.055 $\mu$g/ml to 0.84 $\mu$g/ml of Doxorubicin and about 9.812 $\mu$g/ml to 122.65 $\mu$g/ml of Glimepiride.

In another particular embodiment, the combination of Doxorubicin and Glimepiride comprise between about 0.2 $\mu$M to 1 $\mu$M of Doxorubicin and between about 40 $\mu$M to 180 $\mu$M of Glimepiride, preferably about 50 $\mu$M to 160 $\mu$M of Glimepiride, which is equivalent of about 0.11 $\mu$g/ml to 0.55 $\mu$g/ml of Doxorubicin and about 19.62 $\mu$g/ml to 88.29 $\mu$g/ml of Glimepiride or about 24.53 $\mu$g/ml to 78.5 $\mu$g/ml of Glimepiride. This combination yielded a 3.1-fold increase in the antitumor activity of the combination therapy compared to Doxorubicin alone.

In another particular embodiment, the combination of Doxorubicin and Glimepiride comprise between about 0.2 $\mu$M to 0.5 $\mu$M of Doxorubicin and between about 20 $\mu$M to 80 $\mu$M of Glimepiride, which is equivalent of about 0.11 $\mu$g/ml to 0.28 $\mu$g/ml of Doxorubicin and about 9.81 $\mu$g/ml to 39.24 $\mu$g/ml of Glimepiride. This combination yielded a 2.7-fold increase in the antitumor activity of the combination therapy compared to Doxorubicin alone.

In another particular embodiment, the seven previous paragraphs relating to the combination of Doxorubicin and Glimepiride are for use against brain cancer, preferably glioma.

In a particular embodiment, the amount of Tolazamide administered to a subject range from 10 $\mu$g/ml to 300 $\mu$g/ml. The molecular weight of Tolazamide is 311.401 g/ml, thus a concentration of 100 $\mu$M or 500 $\mu$M is equivalent to 31.14 $\mu$g/ml and 155,7 $\mu$g/ml.

For example, the concentration of Tolazamide can be about any one of 10 $\mu$g/ml, 12 $\mu$g/ml, 14 $\mu$g/ml, 15 $\mu$g/ml, 16 $\mu$g/ml, 18 $\mu$g/ml, 20 $\mu$g/ml, 25 $\mu$g/ml, 30 $\mu$g/ml, 35 $\mu$g/ml, 40 $\mu$g/ml, 45 $\mu$g/ml, 50 $\mu$g/ml, 55 $\mu$g/ml, 60 $\mu$g/ml, 65 $\mu$g/ml, 70 $\mu$g/ml, 75 $\mu$g/ml, 80 $\mu$g/ml, 85 $\mu$g/ml, 90 $\mu$g/ml, 95 $\mu$g/ml, 100 $\mu$g/ml, 110 $\mu$g/ml, 120 $\mu$g/ml, 125 $\mu$g/ml, 130 $\mu$g/ml, 140 $\mu$g/ml, 150 $\mu$g/ml.

In another particular embodiment, the combination of Doxorubicin and Tolazamide comprise between about 0.2 $\mu$M to 1 $\mu$M of Doxorubicin and between about 50 $\mu$M to 600 $\mu$M of Tolazamide, which is equivalent of about 0.11 $\mu$g/ml to 0.55 $\mu$g/ml of Doxorubicin and about 15.57 $\mu$g/ml to 186.84 $\mu$g/ml of Tolazamide

In a particular embodiment, the amount of Gliclazide administered to a subject range from 10 $\mu$g/ml to 300 $\mu$g/ml. The molecular weight of Gliclazide is 323.412 g/ml, thus a concentration of 100 $\mu$M or 500 $\mu$M is equivalent to 32.34 $\mu$g/ml and 161.71 $\mu$g/ml

For example, the concentration of Gliclazide can be about any one of 10 $\mu$g/ml, 12 $\mu$g/ml, 14 $\mu$g/ml, 15 $\mu$g/ml, 16 $\mu$g/ml, 18 $\mu$g/ml, 20 $\mu$g/ml, 25 $\mu$g/ml, 30 $\mu$g/ml, 35 $\mu$g/ml, 40 $\mu$g/ml, 45 $\mu$g/ml, 50 $\mu$g/ml, 55 $\mu$g/ml, 60 $\mu$g/ml, 65 $\mu$g/ml, 70 $\mu$g/ml, 75 $\mu$g/ml, 80 $\mu$g/ml, 85 $\mu$g/ml, 90 $\mu$g/ml, 95 $\mu$g/ml, 100 $\mu$g/ml, 110 $\mu$g/ml, 120 $\mu$g/ml, 125 $\mu$g/ml, 130 $\mu$g/ml, 140 $\mu$g/ml, 150 $\mu$g/ml, 160 $\mu$g/ml.

In another particular embodiment, the combination of Doxorubicin and Gliclazide comprise between about 0.2 $\mu$M to 1 $\mu$M of Doxorubicin and between about 50 $\mu$M to 600 $\mu$M of Gliclazide, which is equivalent of about 0.11 $\mu$g/ml to 0.55 $\mu$g/ml of Doxorubicin and about 16.17 $\mu$g/ml to 194.04 $\mu$g/ml of Gliclazide

In a particular embodiment, the amount of Chlorpropamide administered to a subject range from 10 $\mu$g/ml to 300 $\mu$g/ml. The molecular weight of Chlorpropamide is 276.74 g/ml, thus a concentration of 100 $\mu$M or 500 $\mu$M is equivalent to 27.67 $\mu$g/ml and 138.37 $\mu$g/ml.

For example, the concentration of Chlorpropamide can be about any one of 10 $\mu$g/ml, 12 $\mu$g/ml, 14 $\mu$g/ml, 15 $\mu$g/ml, 16 $\mu$g/ml, 18 $\mu$g/ml, 20 $\mu$g/ml, 25 $\mu$g/ml, 30 $\mu$g/ml, 35 $\mu$g/ml, 40 $\mu$g/ml, 45 $\mu$g/ml, 50 $\mu$g/ml, 55 $\mu$g/ml, 60 $\mu$g/ml, 65 $\mu$g/ml, 70 $\mu$g/ml, 75 $\mu$g/ml, 80 $\mu$g/ml, 85 $\mu$g/ml, 90 $\mu$g/ml, 95 $\mu$g/ml, 100 $\mu$g/ml, 110 $\mu$g/ml, 120 $\mu$g/ml, 125 $\mu$g/ml, 130 $\mu$g/ml.

In another particular embodiment, the combination of Doxorubicin and Chlorpropamide comprise between about 0.2 $\mu$M to 1 $\mu$M of Doxorubicin and between about 50 $\mu$M to 600 $\mu$M of Chlorpropamide, which is equivalent of about 0.11 $\mu$g/ml to 0.55 $\mu$g/ml of Doxorubicin and about 13.84 $\mu$g/ml to 166.02 $\mu$g/ml of Chlorpropamide

In a particular embodiment, the amount of Tolbutamide administered to a subject range from 10 $\mu$g/ml to 300 $\mu$g/ml. The molecular weight of Tolbutamide is 270.35 g/ml, thus a concentration of 100 $\mu$M or 500 $\mu$M is equivalent to 27.04 $\mu$g/ml and 135.18 $\mu$g/ml

For example, the concentration of Tolbutamide can be about any one of 10 μg/ml, 12 μg/ml, 14 μg/ml, 15 μg/ml, 16 μg/ml, 18 μg/ml, 20 μg/ml, 25 μg/ml, 30 μg/ml, 35 μg/ml, 40 μg/ml, 45 μg/ml, 50 μg/ml, 55 μg/ml, 60 μg/ml, 65 μg/ml, 70 μg/ml, 75 μg/ml, 80 μg/ml, 85 μg/ml, 90 μg/ml, 95 μg/ml, 100 μg/ml, 110 μg/ml, 120 μg/ml, 125 μg/ml, 130 μg/ml.

In another particular embodiment, the combination of Doxorubicin and Tolbutamide comprise between about 0.2 μM to 1 μM of Doxorubicin and between about 50 μM to 600 μM of Tolbutamide, which is equivalent of about 0.11 μg/ml to 0.55 μg/ml of Doxorubicin and about 13.52 μg/ml to 162.24 μg/ml of Tolbutamide

In a particular embodiment, the amount of Glipizide administered to a subject range from 10 μg/ml to 300 μg/ml. The molecular weight of Glipizide is 445,536 g/ml, thus a concentration of 100 μM or 500 μM is equivalent to 44.55 μg/ml and 222.77 μg/ml

For example, the concentration of Glipizide can be about any one of 45 μg/ml, 50 μg/ml, 55 μg/ml, 60 μg/ml, 65 μg/ml, 70 μg/ml, 75 μg/ml, 80 μg/ml, 85 μg/ml, 90 μg/ml, 95 μg/ml, 100 μg/ml, 110 μg/ml, 120 μg/ml, 125 μg/ml, 130 μg/ml, 140 μg/ml, 150 μg/ml, 160 μg/ml, 170 μg/ml, 175 μg/ml, 180 μg/ml, 190 μg/ml, 200 μg/ml, 220 μg/ml.

In another particular embodiment, the combination of Doxorubicin and Glipizide comprise between about 0.2 μM to 1 μM of Doxorubicin and between about 50 μM to 600 μM of Glipizide, which is equivalent of about 0.11 μg/ml to 0.55 μg/ml of Doxorubicin and about 22.28 μg/ml to 267.3 μg/ml of Glipizide.

In a particular embodiment, the amount of Docetaxel administered to a subject range from 0.001 μg/ml to 10 μg/ml. The molecular weight of Docetaxel is 807,879 g/mol, thus a concentration of 0.1 μM or 1 μM is equivalent to 0.081 μg/ml and 0.81 μg/ml
The concentration of Docetaxel can be about any one of 0.001 μg/ml, 0.002 μg/ml, 0.004 μg/ml, 0.006 μg/ml, 0.008 μg/ml, 0.01 μg/ml, 0.015 μg/ml, 0.02 μg/ml, 0.025 μg/ml, 0.03 μg/ml, 0.035 μg/ml, 0.04 μg/ml, 0.045 μg/ml, 0.05 μg/ml, 0.055 μg/ml, 0.06 μg/ml, 0.065 μg/ml, 0.07 μg/ml, 0.075 μg/ml, 0.08 μg/ml, 0.085 μg/ml, 0.09 μg/ml, 0.095 μg/ml, 0.1 μg/ml, 0.2μg/ml, 0.3μg/ml, 0.4 μg/ml, 0.5 μg/ml, 0.6μg/ml, 0.7μg/ml, 0.8 μg/ml, 0.9 μg/ml, 0.10 μg/ml, 0.12 μg/ml, 0.14 μg/ml, 0.15 μg/ml, 0.16 μg/ml, 0.18 μg/ml, 0.20 μg/ml, 0.25 μg/ml, 0.30 μg/ml, 0.35 μg/ml, 0.40 μg/ml, 0.45 μg/ml, 0.50 μg/ml, 0.55 μg/ml, 0.60 μg/ml, 0.65 μg/ml, 0.70 μg/ml, 0.75 μg/ml, 0.80 μg/ml, 0.85 μg/ml, 0.90 μg/ml, 0.95 μg/ml, 1 μg/ml, 1.10 μg/ml, 1.20 μg/ml, 1.25 μg/ml, 1.30 μg/ml, 1.40 μg/ml, 1.50 μg/ml, 1.60 μg/ml, 1.70 μg/ml, 1.75 μg/ml, 1.80 μg/ml, 1.90 μg/ml, 2.00 μg/ml, 2.20 μg/ml, 2.40 μg/ml, 2.50 μg/ml, 2.60 μg/ml, 2.80 μg/ml, 3. pg/ml, 3.20 μg/ml, 3.40 μg/ml, 3.50 μg/ml, 3.60 μg/ml, 3.80 μg/ml, 4.0 μg/ml, 4.20 μg/ml, 4.40 μg/ml, 4.50 μg/ml, 4.60 μg/ml, 4.80 μg/ml, or 5.00 μg/ml, 5.5 μg/ml, 6.0 μg/ml, 6.5 μg/ml, 7.0 μg/ml, 7.5 μg/ml, 8.0 μg/ml, 8.5 μg/ml, 9.0 μg/ml, 9.5 μg/ml, 10 μg/ml.
In another particular embodiment, the combination of Docetaxel and Glimepiride comprise between about 0.01 μM to 0.5 μM of Docetaxel and between about 20 μM to 500 μM of Glimepiride, which is equivalent of about 0.0081 μg/ml to 0.405 μg/ml of Docetaxel and about 9.812 μg/ml to 245.30 μg/ml of Glimepiride.
In another particular embodiment, Glimepiride at a concentration of about 20 μM to 500 μM, can be replaced by a Glimepiride analog selected from: Tolazamide, Gliclazide, Chlorpropamide, Tolbutamide and Glipizide, at a concentration of about 20 μM to 500 μM
In another particular embodiment, the above paragraph relating to the combination of Docetaxel and Glimepiride are for use against, breast cancer, lung cancer or brain cancer.
In a particular embodiment, the amount of Capecitabine administered to a subject range from 0.01 μg/ml to 10 μg/ml. The molecular weight of Capecitabine is 359,35 g/mol, thus a concentration of 0.1 μM or 1 μM is equivalent to 0.036 μg/ml and 0.36 μg/ml
The concentration of Capecitabine can be about any one of 0.001 μg/ml, 0.002 μg/ml, 0.004 μg/ml, 0.006 μg/ml, 0.008 μg/ml, 0.01 pg/ml, 0.015 μg/ml, 0.02 μg/ml, 0.025 μg/ml, 0.03 μg/ml, 0.035 μg/ml, 0.04 μg/ml, 0.045 μg/ml, 0.05 μg/ml, 0.055 μg/ml, 0.06 μg/ml, 0.065 μg/ml, 0.07 μg/ml, 0.075 μg/ml, 0.08 μg/ml, 0.085 μg/ml, 0.09 μg/ml, 0.095 μg/ml, 0.1 μg/ml, 0.2μg/ml, 0.3μg/ml, 0.4 μg/ml, 0.5 μg/ml, 0.6μg/ml, 0.7μg/ml, 0.8 μg/ml, 0.9 μg/ml, 0.10 μg/ml, 0.12 μg/ml, 0.14 μg/ml, 0.15 μg/ml, 0.16 μg/ml, 0.18 μg/ml, 0.20 μg/ml, 0.25 μg/ml, 0.30 μg/ml, 0.35 μg/ml, 0.40 μg/ml, 0.45 μg/ml, 0.50 μg/ml, 0.55 μg/ml, 0.60 μg/ml, 0.65 μg/ml, 0.70 μg/ml, 0.75 μg/ml, 0.80 μg/ml, 0.85 μg/ml, 0.90 μg/ml, 0.95 μg/ml, 1 μg/ml, 1.10 μg/ml, 1.20 μg/ml, 1.25 μg/ml, 1.30 μg/ml, 1.40 μg/ml, 1.50 μg/ml, 1.60 μg/ml, 1.70 μg/ml, 1.75 μg/ml, 1.80 μg/ml, 1.90 μg/ml, 2.00 μg/ml, 2.20 μg/ml, 2.40 μg/ml, 2.50 μg/ml, 2.60 μg/ml, 2.80 μg/ml, 3. μg/ml, 3.20 μg/ml, 3.40 μg/ml, 3.50 μg/ml, 3.60 μg/ml, 3.80 μg/ml, 4.0 μg/ml, 4.20 μg/ml, 4.40 μg/ml, 4.50 μg/ml, 4.60 μg/ml, 4.80 μg/ml, or 5.00 μg/ml, 5.5 μg/ml, 6.0 μg/ml, 6.5 μg/ml, 7.0 μg/ml, 7.5 μg/ml, 8.0 μg/ml, 8.5 μg/ml, 9.0 μg/ml, 9.5 μg/ml, 10 μg/ml.
In another particular embodiment, the combination of Capecitabine and Glimepiride comprise between about 0.01 μM to 0.5 μM of Capecitabine and between about 20 μM to 500 μM of Glimepiride, which is equivalent of about 0.0036 μg/ml to 0.18 μg/ml of Capecitabine and about 9.812 μg/ml to 245.30 μg/ml of Glimepiride.
In another particular embodiment, the above paragraph relating to the combination of Capecitabine and Glimepiride are for use against, breast cancer, lung cancer or brain cancer.

In another particular embodiment, Glimepiride at a concentration of about 20 µM to 500 µM, can be replaced by a Glimepiride analog selected from: Tolazamide, Gliclazide, Chlorpropamide, Tolbutamide and Glipizide, at a concentration of about 20 µM to 500 µM

According to the invention the composition may comprise between 20% - 99.5% by weight of the combination of the anticancer agent selected from the group consisting of: Doxorubicin, Docetaxel and Capecitabine, preferably Doxorubicinand Glimepiride and/or a Glimepiride analog, particularly 25%-95% by weight of said combination and more particularly 50% - 95% by weight of said combination.

Another object of the invention relates to a pharmaceutical composition comprising the synergistic composition described above of an anticancer agent selected from the group consisting of: Doxorubicin, Docetaxel and Capecitabine and

Glimepiride or

a Glimepiride analog selected from the group consisting of Gliquidone, Tolazamide, Gliclazide, Chlorpropamide, Tolbutamide and Glipizide plus:

- one or more additional active compound, and/or

- one or more pharmaceutically acceptable carrier, adjuvant, or vehicle

for use in the treatment of cancer

In a particular embodiment, the anticancer agent is Doxorubicin

In another particular embodiment the synergistic composition comprises Docetaxel and Glimepiride.

In another particular embodiment the synergistic composition comprises Capecitabine and Glimepiride.

In another particular embodiment, the one or more additional active compound is selected from Capecitabine and Docetaxel

The pharmaceutically acceptable carrier(s) adjuvant, or vehicle must be acceptable in the sense of being compatible with the other ingredients of the formula. In embodiments of the invention, the composition comprises a pharmaceutically acceptable carrier adjuvant, or vehicle selected to be compatible with glimepiride, or glimepiride analogs and compatible with an anticancer agent selected from the group consisting of: Doxorubicin, Docetaxel and Capecitabine.

Also disclosed herein are pharmaceutical compositions comprising at least one of the compounds disclosed herein and/or at least one pharmaceutically acceptable salt thereof, which compositions may further include at least one additional active pharmaceutical ingredient and/or at least one carrier. Also disclosed are methods of treating the Betacoronavirus -mediated infection comprising administering at least one of the compounds disclosed herein and/or at least one pharmaceutically acceptable salt thereof, optionally as part of a pharmaceutical composition comprising at least one additional component, to a subject in need thereof.

Non-limiting examples of pharmaceutically acceptable salts derived from appropriate acids include: salts formed with inorganic acids, such as hydrochloric acid, hydrobromic acid, phosphoric acid, sulfuric acid, or perchloric acid; salts formed with organic acids, such as acetic acid, oxalic acid, maleic acid, tartaric acid, citric acid, succinic acid or malonic acid; and salts formed by using other methods used in the art, such as ion exchange. Non-limiting examples of pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, and valerate salts. Pharmaceutically acceptable salts derived from appropriate bases include alkali metal, alkaline earth metal, ammonium, and N+ (C1-4 alkyl)4 salts. This disclosure also envisions the quaternization of any basic nitrogen-containing groups of the compounds disclosed herein. Suitable non-limiting examples of alkali and alkaline earth metal salts include salts of sodium, lithium, potassium, calcium, and magnesium. Further non-limiting examples of pharmaceutically acceptable salts include salts of ammonium, quaternary ammonium, and amine cations formed using counterions such as halide, hydroxide, carboxylate, sulfate, phosphate, nitrate, lower alkyl sulfonate and aryl sulfonate. Other suitable, non-limiting examples of pharmaceutically acceptable salts include besylate and glucosamine salts.

As described above, the pharmaceutically compositions of the present invention additionally can comprise one or more pharmaceutically acceptable carrier, adjuvant, or vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutically acceptable composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, glycine, sorbic acid, or potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

In a particular embodiment, a skilled person would know which patients are hypersensitive to any of the drugs of the combination of the present invention in order to adjust the dosage. For example: Marked persistent myelosuppression induced by prior treatment with other antitumor agents or by radiotherapy, severe hepatic impairment, severe myocardial insufficiency, recent myocardial infarction, severe arrhythmias, history of severe cardiac disease, previous treatment with maximum cumulative doses of doxorubicin, daunorubicin, epirubicin, idarubicin and/or other anthracyclines and anthracenediones and Hepatic Dysfunction: doxorubicin hydrochloride injection dosage must be reduced if the bilirubin is elevated as follows: Serum Bilirubin 1.2-3.0 mg/dL - give 1/2 of recommended starting dose, > 3 mg/dL - give 1/4 of recommended starting dose. Doxorubicin should not be administered to patients with severe hepatic impairment

Furthermore, for Special Populations: Lower starting doses or longer intervals between cycles may need to be considered for heavily pretreated patients, children, elderly patients, obese patients, or patients with neoplastic bone marrow infiltration

The pharmaceutical composition of the invention may comprise one or more additional active compound selected from cytosine arabinoside, vincristine, prednisone, L-asparaginase, arabinosyl cytosine, 5-fluorouracil, cyclophosphamide, methotrexate, procarbazine, cisplatinum, etoposide, bleomycin, dacarbazine, and mitomycin-C or combinations thereof

In a particular embodiment, for acute lymphocytic leukemia in adults: the one or more additional active compounds are vincristine and prednisone or with cytosine arabinoside, vincristine and prednisone.

In another particular embodiment for acute lymphocytic leukemia in children: the one or more additional active compounds are L-asparaginase, vincristine and prednisone.

In another particular embodiment for acute non-lymphocytic leukemia: the one or more additional active compounds are cytosine arabinosyl or with arabinosyl cytosine, vincristine and prednisone.

In another particular embodiment for carcinoma of the breast: the one or more additional active compounds are 5-fluorouracil and/or cyclophosphamide or with vincristine with or without cyclophosphamide, or with taxane therapy.

In another particular embodiment for bronchogenic carcinoma, non-small cell: the one or more additional active compounds are cyclophosphamide, methotrexate and procarbazine or with cyclophosphamide and cisplatinum.

In another particular embodiment for bronchogenic carcinoma, small cell: the one or more additional active compounds are vincristine or etoposide (VP-16) and cyclophosphamide.

In another particular embodiment for Hodgkin's disease: the one or more additional active compounds are bleomycin, vincristine and dacarbazine.

In another particular embodiment for Non-Hodgkin's lymphoma: the one or more additional active compounds are cyclophosphamide, vincristine and prednisone, or bleomycin, cyclophosphamide, vincristine and prednisone.

In another particular embodiment for Carcinoma of the ovary: the additional active compound is cisplatinum.

In another particular embodiment for Soft tissue sarcoma: the one or more additional active compounds are dacarbazine, or with dacarbazine, cyclophosphamide and vincristine.

In another particular embodiment for Carcinoma of the bladder: the one or more additional active compounds are methotrexate, vinblastine and cisplatinum or cisplatinum and cyclophosphamide or with 5-fluorouracil.

In another particular embodiment for Carcinoma of the stomach: the one or more additional active compounds are 5-fluorouracil and mitomycin-C.

The compounds and pharmaceutical compositions described herein can be administered to the subject in a number of ways depending on whether local or systemic treatment is desired, and on the area to be treated. Thus, for example, a compound or pharmaceutical composition described herein can be administered as an ophthalmic solution and/or ointment to the surface of the eye. Moreover, a compound or pharmaceutical composition can be administered to a subject vaginally, rectally, intranasally, orally, by inhalation, or parenterally, for example, by intradermal, subcutaneous, intramuscular, intraperitoneal, intrarectal, intraarterial, intralymphatic, intravenous, intrathecal and intratracheal routes. Parenteral administration, if used, is generally characterized by injection. Injectables can be prepared in conventional forms, either as liquid solutions or suspensions, solid forms suitable for solution or suspension in liquid prior to injection, or as emulsions. A more recently revised approach for parenteral administration involves use of a slow release or sustained release system such that a constant dosage is maintained.

In a particular embodiment, when Glimepiride, or Glimepiride analogs are administered before an anticancer agent selected from the group consisting of: Doxorubicin, Docetaxel and Capecitabine in order to reach the appropriate concentration in the body, subsequently, the administration of an anticancer agent selected from the group consisting of: Doxorubicin, Docetaxel and Capecitabine or a mixture of Glimepiride or Glimepiride analogs and an anticancer agent selected from the group consisting of: Doxorubicin, Docetaxel and Capecitabine will be done intravenously.

In another particular embodiment, when the combination is administered simultaneously, it will be done intravenously

As used herein, the phrase "and/or" when used in a list of two or more items, means that any one of the listed items can be employed by itself or any combination of two or more of the listed items can be employed. For example, if a composition is described as containing or excluding components A, B, and/or C, the composition can contain or exclude A alone; B alone; C alone; A and B in combination; A and C in combination; B and C in combination; or A, B, and C in combination.

The present description also uses numerical ranges to quantify certain parameters relating to various embodiments of the invention. It should be understood that when numerical ranges are provided, such ranges are to be construed as providing literal Support for claim limitations that only recite the lower value of the range as well as claim limitations that only recite the upper value of the range. For example, a disclosed numerical range of about 10 to about 100 provides literal support for a claim reciting "greater than about 10 (with no upper bounds) and a claim reciting "less than about 100 (with no lower bounds).

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1 , about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below

**BRIEF DESCRIPTION OF FIGURES:**

FIGURE 1. Structure of the Glimepiride molecule.

FIGURE 2. Structure of the Doxorubicin molecule.

FIGURE 3. Common structural core for benzenesulfonylureas.

FIGURE 4. Chemical structures and names of sulfonylurea-based antidiabetic drugs.

**FIGURE 5.** Cytotoxic effects of various doses of Glimepiride on MCF-7 cells. The table shows the fraction of viable cells after treatment relative to untreated controls.

**FIGURE 6.** Cytotoxic effects of various doses of Doxorubicin on MCF-7 cells. The table shows the fraction of viable cells after treatment relative to untreated controls.

**FIGURE 7.** Cytotoxic effects of combinations of different doses of Doxorubicin (0.225, 0.450 and 0.900 $\mu$M) and Glimepiride (100, 200, 300, 400 and 500 $\mu$M) on MCF-7 cells. The left side of the table shows the fraction of viable cells after treatment relative to the untreated control. The right side of the table shows the combination index value for the corresponding drug combination.

**FIGURE 8.** Comparison of the cytotoxicity of combinations of different doses of Doxorubicin (0.225, 0.450 and 0.900 $\mu$M) and Glimepiride (100, 200, 300, 400 and 500 $\mu$M) against MCF-7 cells with the cytotoxicity of the corresponding dose of Doxorubicin alone.

**FIGURE 9.** Increase in cytotoxicity for combinations of various doses of Doxorubicin (0.225, 0.450 and 0.900 $\mu$M) and Glimepiride (100, 200, 300, 400 and 500 $\mu$M) against MCF-7 cells compared to cytotoxicity with the corresponding dose of Doxorubicin alone (taken as 100%).

**FIGURE 10.** Cytotoxic effects of combinations of different doses of Doxorubicin (0.113, 0.225 and 0.450 $\mu$M) and Glimepiride (46.6, 93.1 and 186.2 $\mu$M) on MCF-7 cells. The left side of the table shows the fraction of viable cells after treatment relative to the untreated control. The right side of the table shows the combination index value for the corresponding drug combination.

**FIGURE 11.** Comparison of the cytotoxicity of combinations of different doses of Doxorubicin (0.113, 0.225 and 0.450 $\mu$M) and Glimepiride (46.6, 93.1 and 186.2 $\mu$M) against MCF-7 cells with the cytotoxicity of the corresponding dose of Doxorubicin alone.

**FIGURE 12.** Increase in cytotoxicity for combinations of various doses of Doxorubicin (0.113, 0.225 and 0.450 $\mu$M) and Glimepiride (46.6, 93.1 and 186.2 $\mu$M) against MCF-7 cells compared to cytotoxicity with the corresponding dose of Doxorubicin alone (taken as 100%).

**FIGURE 13.** Cytotoxic effects of various doses of Glimepiride on A549 cells. The table shows the fraction of viable cells after treatment relative to untreated controls.

**FIGURE 14.** Cytotoxic effects of various doses of Glimepiride on A549 cells. The table shows the fraction of viable cells after treatment relative to untreated controls.

**FIGURE 15.** Cytotoxic effects of combinations of different doses of Doxorubicin (0.145, 0.290 and 0.580 $\mu$M) and Glimepiride (100, 200, 300, 400 and 500 $\mu$M) on A549 cells. The left side of the table shows the fraction of viable cells after treatment relative to the untreated control. The right side of the table shows the combination index value for the corresponding drug combination.

**FIGURE 16.** Comparison of the cytotoxicity of combinations of different doses of Doxorubicin (0.145, 0.290 and 0.580 $\mu$M) and Glimepiride (100, 200, 300, 400 and 500 $\mu$M) against A549 cells with the cytotoxicity of the corresponding dose of Doxorubicin alone.

**FIGURE 17.** Increase in cytotoxicity for combinations of various doses of Doxorubicin (0.145, 0.290 and 0.580 $\mu$M) and Glimepiride (100, 200, 300, 400 and 500 $\mu$M) against A549 cells compared to cytotoxicity with the corresponding dose of Doxorubicin alone (taken as 100%).

**FIGURE 18.** Cytotoxic effects of combinations of different doses of Doxorubicin (0.073, 0.145 and 0.290 $\mu$M) and Glimepiride (95.1, 190.3 and 380.5 $\mu$M) on A549 cells. The left side of the table shows the fraction of viable cells after treatment relative to the untreated control. The right side of the table shows the combination index value for the corresponding drug combination.

**FIGURE 19.** Comparison of the cytotoxicity of combinations of different doses of Doxorubicin (0.073, 0.145 and 0.290 $\mu$M) and Glimepiride (95.1, 190.3 and 380.5 $\mu$M) against A549 cells with the cytotoxicity of the corresponding dose of Doxorubicin alone.

**FIGURE 20.** Increase in cytotoxicity for combinations of various doses of Doxorubicin (0.073, 0.145 and 0.290 $\mu$M) and Glimepiride (95.1, 190.3 and 380.5 $\mu$M) against A549 cells compared to cytotoxicity with the corresponding dose of Doxorubicin alone (taken as 100%).

**FIGURE 21.** Cytotoxic effects of various doses of Glimepiride on U-87MG cells. The table shows the fraction of viable cells after treatment relative to untreated controls.

**FIGURE 22.** Cytotoxic effects of various doses of Glimepiride on U-87MG cells. The table shows the fraction of viable cells after treatment relative to untreated controls.

**FIGURE 23.** Cytotoxic effects of combinations of various doses of Doxorubicin (0.52, 1.04 and 2.08 $\mu$M) and Glimepiride (100, 200, 300, 400 and 500 $\mu$M) on U-87MG cells. The left side of the table shows the fraction of viable cells after treatment relative to the untreated control. The right side of the table shows the combination index value for the corresponding drug combination.

**FIGURE 24.** Comparison of the cytotoxicity of combinations of various doses of Doxorubicin (0.52, 1.04 and 2.08 $\mu$M) and Glimepiride (100, 200, 300, 400 and 500 $\mu$M) against U-87MG cells with the cytotoxicity of the corresponding dose of Doxorubicin alone.

**FIGURE 25.** Increase in cytotoxicity for combinations of various doses of Doxorubicin (0.52, 1.04 and 2.08 $\mu$M) and Glimepiride (100, 200, 300, 400 and 500 $\mu$M) against U-87MG cells compared to cytotoxicity with the corresponding dose of Doxorubicin alone (taken as 100%).

**FIGURE 26.** Cytotoxic effects of combinations of different doses of Doxorubicin (0.26, 0.52 and 1.04 $\mu$M) and Glimepiride (41.2, 82.4 and 164.8 $\mu$M) on U-87MG cells. The left side of the table shows the fraction of viable cells after treatment relative to the untreated control. The right side of the table shows the combination index value for the corresponding drug combination.

**FIGURE 27.** Comparison of the cytotoxicity of combinations of various doses of Doxorubicin (0.26, 0.52 and 1.04 $\mu$M) and Glimepiride (41.2, 82.4, and 164.8 $\mu$M) against U-87MG cells with the cytotoxicity of the corresponding dose of Doxorubicin alone.

**FIGURE 28.** Increase in cytotoxicity for combinations of various doses of Doxorubicin (0.26, 0.52 and 1.04 $\mu$M) and Glimepiride (41.2, 82.4 and 164.8 $\mu$M) against U-87MG cells compared to cytotoxicity with the corresponding dose of Doxorubicin alone (taken as 100%).

**FIGURE 29.** Cytotoxic effects of different doses of Glimepiride analogs on MCF-7 cells. The table shows the fraction of viable cells after treatment relative to untreated controls. The abbreviations used refer to the following drugs: GLIQ - Gliquidone, TOLA - Tolazamide, GLIC - Gliclazide, CHLO - Chlorpropamide, GLIB - Glibenclamide, TOLB - Tolbutamide, GLIP - Glipizide.

**FIGURE 30.** Comparison of cytotoxicity of various doses of Glimepiride analogs on MCF-7 cells. The graph shows the fraction of viable cells after treatment versus untreated control. The abbreviations used refer to the following drugs: GLIQ - Gliquidone, TOLA - Tolazamide, GLIC - Gliclazide, CHLO - Chlorpropamide, GLIB - Glibenclamide, TOLB - Tolbutamide, GLIP - Glipizide

**FIGURE 31.** Summary of the cytotoxicity of combinations of different doses of Doxorubicin (0.225, 0.450 and 0.900 $\mu$M) and Glimepiride analogs (100, 200, 300, 400 and 500 $\mu$M) on MCF-7 cells. The left side of the table shows the decrease in the number of viable cells after treatment compared to untreated controls. The right side of the table shows the combination index value for the corresponding drug combination. The abbreviations used refer to the following drugs: DOXO - Doxorubicin, GLIQ - Gliquidone, TOLA - Tolazamide, GLIC - Gliclazide, CHLO - Chlorpropamide, GLIB - Glibenclamide, TOLB - Tolbutamide, GLIP - Glipizide.

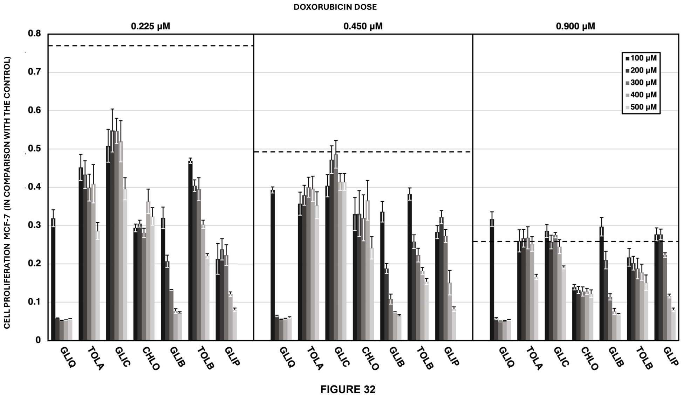

**FIGURE 32.** Effect of the combination of Doxorubicin (0.225, 0.450 and 0.900 $\mu$M) and Glimepiride analogs (100, 200, 300, 400 and 500 $\mu$M) at different doses of both drugs on MCF-7 cells. The dashed line indicates the fraction of viable cells after treatment with the corresponding dose of Doxorubicin alone. All results below the dashed line indicate higher antiproliferative activity of the Doxorubicin-drug combination against MCF-7 cells than Doxorubicin alone. The abbreviations used refer to the following drugs: DOXO - Doxorubicin, GLIQ - Gliquidone, TOLA - Tolazamide, GLIC - Gliclazide, CHLO - Chlorpropamide, GLIB - Glibenclamide, TOLB - Tolbutamide, GLIP - Glipizide.

**FIGURE 33.** Increased cytotoxicity for combinations of different doses of Doxorubicin (0.225, 0.450 and 0.900 μM) and Glimepiride analogs (100, 200, 300, 400 and 500 μM) against MCF-7 cells compared to cytotoxicity with the corresponding dose of Doxorubicin alone (taken as 100%). The abbreviations used refer to the following drugs: DOXO - Doxorubicin, GLIQ - Gliquidone, TOLA - Tolazamide, GLIC - Gliclazide, CHLO - Chlorpropamide, GLIB - Glibenclamide, TOLB - Tolbutamide, GLIP - Glipizide.

## EXPERIMENTAL PROCEDURES

For *in vitro* experiments, Doxorubicin and Glimepiride were purchase from Sigma-Aldrich (Taufkirchen, Germany). Before each experiment, Doxorubicin was freshly dissolved in distilled water to ensure consistent formulation quality. Glimepiride and its analogs were first prepared as a stock solution in DMSO, and then the drugs were dissolved fresh in culture medium enriched with Tween 80 (final concentration 0.05%).

Human cancer cells (MCF-7, A549 and U-87MG) and normal endothelial cells (HMEC-1) were obtained from American Type Cell Culture (Rockville, MD). The cancer cell lines (MCF-7, A549 and U-87MG) were cultured as monolayers in Dulbecco's modified Eagle's medium supplemented with 10% (v/v) fetal bovine serum and 1% (v/v) penicillin/streptomycin mixture (10,000 U/mL penicillin and 10 mg/mL streptomycin) in a 100% humidified atmosphere of 5% $CO_2$ and 95% air at 37 °C. Normal endothelial cells (HMEC-1) were cultured in MCDB 131 medium supplemented with 10% (v/v) fetal bovine serum, 10 ng/mL EGF, 1 μg/mL hydrocortisone and 1% (v/v) penicillin/streptomycin solution, in a humidified atmosphere of 5% $CO_2$ and 95% air at 37 °C. Cell cultures were kept in the exponential growth phase by regularly passaging cells three times a week with 0.25% trypsin/EDTA mixture.

The cytotoxic activity of the test compounds against cancer cell lines was tested using the standard MTT (3-(4,5-dimethylthiazol-2-yl)-2,5-diphenyltetrazolium bromide) colorimetric assay. Cytotoxic activity against endothelial cells was tested by an analogous method using the XTT (2,3-bis-(2-methoxy-4-nitro-5-sulfophenyl)-2H-tetrazolium-5-carboxanilide) colorimetric assay. Briefly, exponentially proliferating cells were seeded into each well of a 96-well microplate (about $5 \times 10^3$ cells/well). After 24 hours, test compounds at various concentrations were added to the respective wells, and the cells were incubated for another 72 hours. After incubating the cells with the test compounds, the medium was removed and MTT solution with a final concentration of 0.5 mg/mL was added. After 4 hours of incubation, the MTT solution was replaced with DMSO to dissolve the resulting violet formazan crystals in metabolically viable cells. Plates were gently shaken at room temperature until the formazan was completely dissolved and read at 540 nm using a microplate reader. With the XTT method, there is no dissolution step in DMSO (XTT is reduced to a highly water-soluble, orange product instead of the insoluble formazan), and the plate is read at 450 nm. The experiment was repeated at least three times under the same conditions. The cytotoxicity of the test compounds was evaluated based on their $IC_{50}$ values, i.e., the concentration causing a 50% decrease in cell viability compared to untreated (control) cells, which was arbitrary taken as 100%. $IC_{50}$ values were calculated using GraphPad Prism software. To test the synergistic effect in the two-drug studies, the same methodology was used as in the single-drug studies, using MTT and XTT colorimetric assays. The test drugs were administered to the cells at the same time.

The combination index (CI) method - a dimensionless quantity used to determine and quantify the type of drug interaction - for binary combinations was calculated using CompuSyn software based on the equation:

$$(CI)_x = \frac{(D)_1}{(D_x)_1} + \frac{(D)_2}{(D_x)_2} = \frac{(D)_1}{(D_m)_1 \left[ f_a \big/ (1-f_a) \right]^{\frac{1}{m_1}}} + \frac{(D)_2}{(D_m)_2 \left[ f_a \big/ (1-f_a) \right]^{\frac{1}{m_2}}}$$

Where $(D_X)_1$ is the dose of drug $D_1$ that alone inhibits cell growth by x%, $(D_X)_2$ is the dose of drug $D_2$ that alone inhibits cell growth by x%, $(D)_1$ and $(D)_2$ are the doses of drugs $D_1$ and $D_2$ that in combination inhibit cell growth by x%. This equation defines "Synergism" (CI < 1), "Additive effect" (CI = 1) and "Antagonism" (CI > 1). This document uses the more precise designations (Table 1) introduced by Chou later:

| Rango del indice de combinación (CI) | Descripción |
|---|---|
| CI<0.10 | Sinergia muy fuerte |
| 0.10<CI<0.30 | Fuerte sinergia |

(continued)

| Rango del indice de combinación (CI) | Descripción |
|---|---|
| 0.30<CI<0.70 | Synergia |
| 0.70<CI<0.85 | Sinergia moderada |
| 0.85<CI<0.95 | Ligera sinergia |
| 0.95<CI<1.10 | Casi aditivo |
| 1.10<CI<1.20 | Ligero antagonismo |
| 1.20<CI<1.45 | Antagonismo moderado |
| 1.45<CI<3.30 | Antagonismo |
| 3.30<CI<10.0 | Fuerte antagonismo |
| CI>10.0 | Antagonismo muy fuerte |

The above definitions of synergism and antagonism in drug combination studies has been proved mathematically by the derivation of unified general Pharmacodynamics and Biodynamics of the Mass-Action Law (T.C. Chou, Pharmacol. Rev. 58 (2006) 621-681; T.C. Chou, J. Theor. Biol. 59 (1976) 253-276; T.C. Chou, Integr. Biol. 3 (2011) 548-559). The CI method and the definitions of "Synergy", "Additive effect" and "Antagonism" developed by T.C. Chou and P. Talalay are now the most widely used method and definitions of these terms, and the original scientific article introducing this theory (T.C. Chou, P. Talalay, Adv. Enz. Regul. 22 (1984) 27-55) has been cited over 5,000 times in over 1,000 biomedical journals worldwide. The article was described by Elsevier News Release (March 16, 2016) as "making history" and the "gold standard" in the study of drug interactions.

All data are expressed as mean±standard deviation (SD) and presented as a percentage of control (untreated cells) taken as 100%, n=3 (or more). Sample size was estimated for Type I and Type II statistical errors of 0.05 and 0.8, respectively. Normality of the data was checked with the Shapiro-Wilk test, and the homogeneity of variance was checked with the Levene or Brown-Forsythe test. The significance of differences between pairs of means was estimated using one-way ANOVA and Tukey's post-hoc test. For each analysis, the post-hoc power of the used tests was checked. Statistical power below 80% was considered a null result, and constructive conclusions were not made. All statistics were calculated using STATISTICA (StatSoft, USA) or GraphPad Prism software.

## EXAMPLES

The following examples set forth methods in accordance with the invention. It is to be understood, however, that these examples are provided by way of illustration, and nothing therein should be taken as a limitation upon the overall scope of the invention.

*Example 1*

The human epithelial cell line (MCF-7), isolated from the breast tissue of a patient with metastatic adenocarcinoma, was tested against Glimepiride and Doxorubicin individually at various doses, and then against a combination therapy containing both agents.

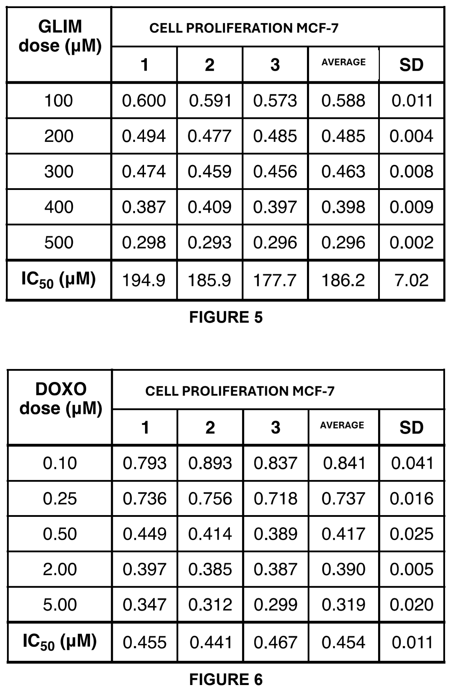

The results are shown in FIGS. 5-9. FIG. 5 shows the dose/response table for Glimepiride alone. FIG. 6 shows the dose/response table for Doxorubicin alone. FIG. 5 shows the mean±standard error for each Glimepiride dose tested. As the concentration of Glimepiride increased, the number of viable cells decreased. The same trend is evident in FIG. 6, which shows the mean±standard error for each Doxorubicin dose tested. The mean $IC_{50}$ concentration, which inhibits MCF-7 cell proliferation by 50% compared to control cells, is 186.2±7.02 $\mu$M and 0.454±0.011 $\mu$M for Glimepiride and Doxorubicin, respectively.

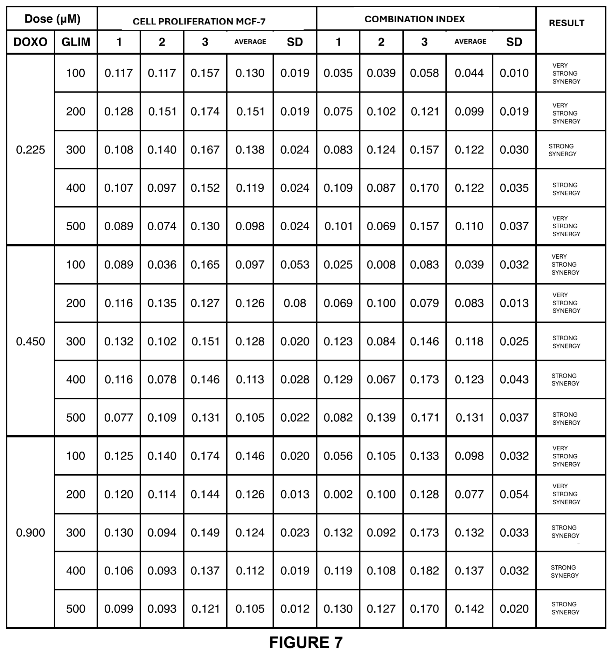

FIG. 7 shows that while both Glimepiride and Doxorubicin alone reduced cell numbers, when combined, their effect was increased, indicating a synergy between Glimepiride and Doxorubicin and chemotherapy beyond their individual additive effects. This study was repeated 3 times, once with increasing doses of Glimepiride (100-500 $\mu$M) plus 0.225 $\mu$M (½ $IC_{50}$) Doxorubicin, once with increasing doses of Glimepiride (100-500 uM) plus 0.45 $\mu$M ($IC_{50}$) Doxorubicin, and once with increasing doses of Glimepiride (100-500 uM) plus 0.90 $\mu$M (2 $IC_{50}$) Doxorubicin. A total of 3 replicates of each combination were performed.

The left side of the table in FIG. 7 (Proliferación de celulas) shows the decrease in the fraction of viable cells (compared to control, which is 1.0) for the same cells when treated with increasing doses of Glimepiride and Doxorubicin.

The right side of the table in FIG. 7 (Índice de combinación) shows the combination index (CI) values. According to the theory of T.C. Chou and P. Talalay, the combination index equation defines each "Synergism" as CI<0.90, "Nearly additive effect" as 0.90<CI<1.10 and each "Antagonism" as CI>1.10. Thus, any combination of Glimepiride and Doxorubicin doses leading to a reduction in the fraction of viable cells that corresponds to CI<0.9 may indicate greater anticancer activity than either drug alone, and therefore synergism between the two. For a more detailed description of the synergistic and antagonistic effect depending on the range of CI values, see the section on experimental methodology.

At each dose of Doxorubicin tested (0.225, 0.45 and 0.90 $\mu$M), the addition of Glimepiride at doses ranging from 100 to 500 $\mu$M increased the anticancer activity of Doxorubicin, which was greater than the activity of Doxorubicin and Glimepiride alone and the sum of their activities. Regardless of the dose of Doxorubicin used, the most favorable CI values were obtained when Glimepiride was co-administered at a dose of 100 $\mu$M. Increasing the dose of Glimepiride (to 200-500 $\mu$M) did not improved CI values or significant reduce cancer cell survival. Thus, at 0.225 $\mu$M Doxorubicin, a statistically significant reduction in cell number was achieved at the lowest dose of Glimepiride tested (100 $\mu$M). At the lowest dose of Doxorubicin tested (0.225 $\mu$M), where chemotherapy alone results in a 23.1% reduction in cancer cell survival, the combination of 0.225 $\mu$M Doxorubicin and 100 $\mu$M Glimepiride yielded a 4.1-fold higher cancer cell killing efficiency (87% dead cancer cells) than Doxorubicin alone.

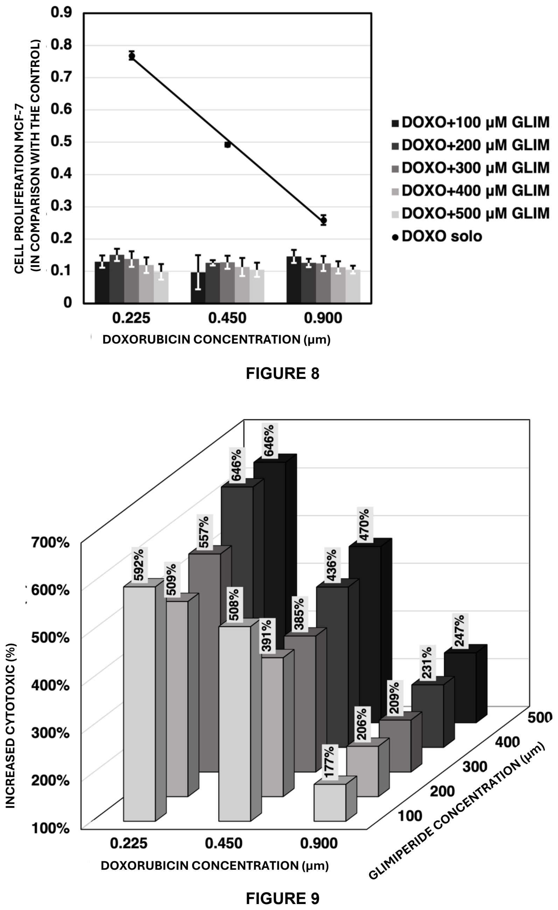

Comparing these results with the graph in FIG. **8,** it can be seen that 0.225 to 0.90 $\mu$M Doxorubicin alone resulted in a smaller reduction in the number of viable cancer cells than any combination of Glimepiride and Doxorubicin. These data support the conclusion that there is a classic synergistic effect between Glimepiride and Doxorubicin. If Doxorubicin and Glimepiride acted through the same mechanism to cause cell death (i.e., binding to the same receptors on cancer cells), then there could be an additive effect.

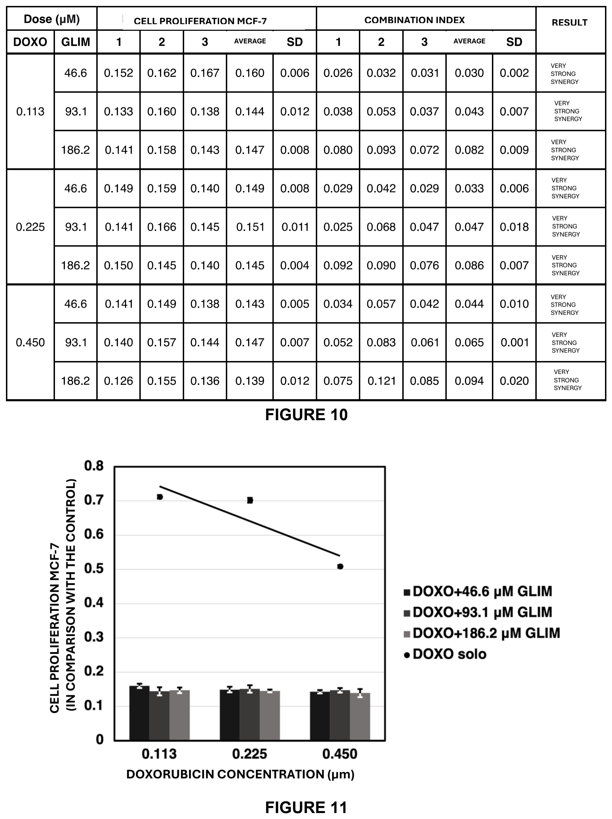

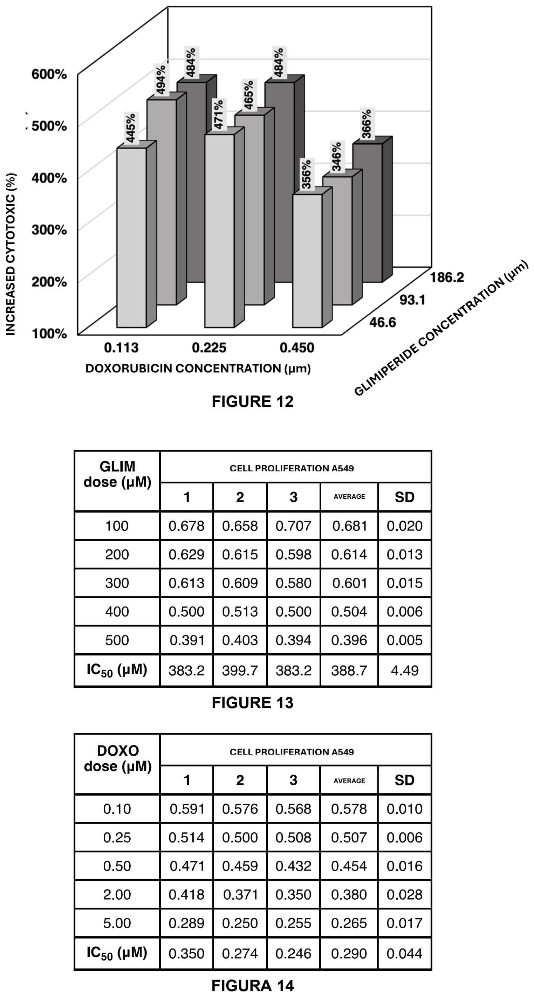

The same studies were repeated using lower doses of Doxorubicin (0.113, 0.225 and 0.450 $\mu$M) and Glimepiride (46.6, 93.1 and 186.2 $\mu$M) corresponding to ¼ $IC_{50}$, ½ $IC_{50}$ and $IC_{50}$, respectively, of both drugs against MCF-7 cells. The results are shown in FIGS. **10-12.** FIG. **10** shows that even in the combination containing lower doses of Doxorubicin and Glimepiride than in Example **1,** there is a very strong synergistic effect between the two drugs. For all Doxorubicin and Glimepiride dose combinations, the CI<1 and ranges from 0.030$\pm$0.002 to 0.094$\pm$0.020, indicating a strong synergistic effect between the drugs against breast cancer cells. As can be seen in FIG. **11,** the strong synergy between Doxorubicin and Glimepiride result in increased anticancer activity of the combination of both drugs against breast cancer cells compared to Doxorubicin alone. FIG. **12** shows that Doxorubicin and Glimepiride dose combinations of Doxorubicin and Glimepiride tested resulted in an approximately 3.5 to 5-fold reduction in MCF-7 cell survival compared to administration of Doxorubicin alone at the appropriate concentration (treated as 100% activity).

The strong synergy of the two drugs resulting in increased anticancer activity od Doxorubicin when Glimepiride is added means that the effective dose of Doxorubicin needed to achieve the same biological effect can be significantly reduced. For example, FIG. **6** shows that Doxorubicin alone administrated at the highest dose tested (5.0 $\mu$M) results in a decrease in MCF-7 cell survival to 31.9%, while the lowest dose of Doxorubicin tested (0.113 $\mu$M) administrated together with the lowest dose of Glimepiride tested (46.6 $\mu$M) results in a decrease in cell survival to 16.0%. This means that by using a more than 40-fold lower dose of Doxorubicin, an almost 3-fold increase in anticancer activity was achieved. Therefore, it can be expected that due to this effect, the dose of Doxorubicin administered to cancer patients can be significantly reduced, preserving its therapeutic effect while causing a reduction in the side effects of therapy with it.

*Example 2*

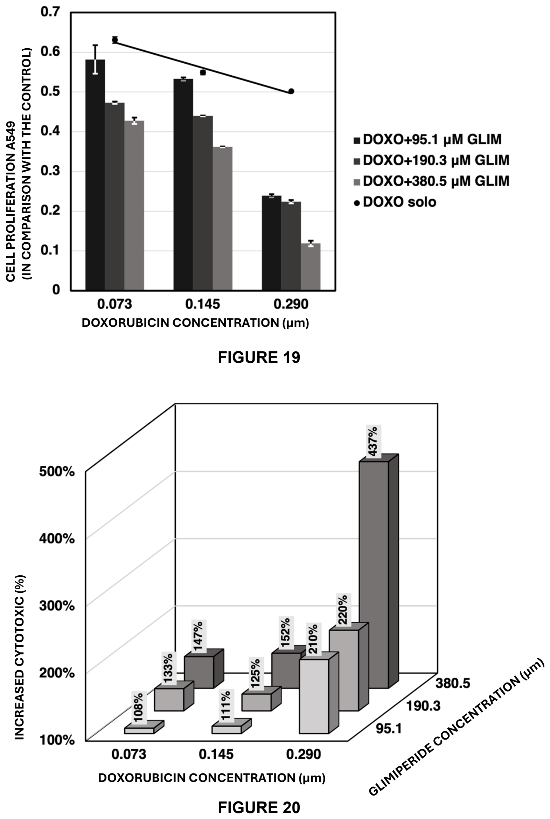

The same studies from Example **1** were repeated, but the MCF-7 cell line was replaced with the A549 cell line, a commonly used human lung adenocarcinoma cell line derived from primary lung cancer. The results are shown in FIGS. **13-20.** As can be seen in FIG. **13,** there is a decrease in cell survival of the A549 line as the Glimepiride dose is increased. The $IC_{50}$ value for cancer cell survival calculated in FIG. **13** is 388.7$\pm$4.49 $\mu$M. FIG. **14** shows that Doxorubicin alone has an $IC_{50}$ value of 0.29$\pm$0.044 $\mu$M.

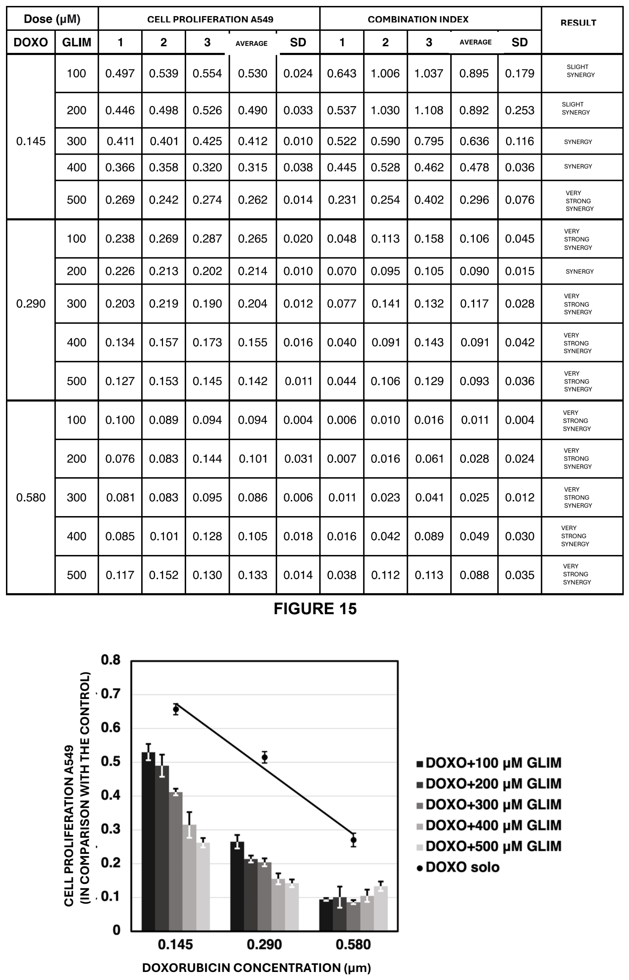

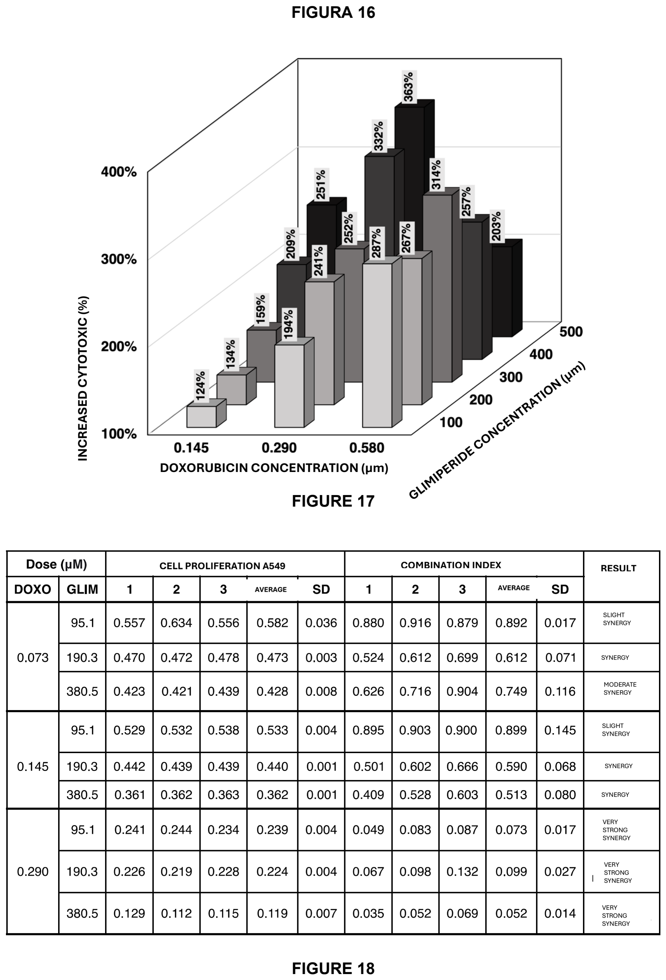

FIG. **15** shows that when Doxorubicin and Glimepiride are administrated together, there is a synergistic effect of any combination of Glimepiride with Doxorubicin on these human lung adenocarcinoma cells. FIG. **16** shows that for all doses of Doxorubicin tested (0.145, 0.29 and 0.58 $\mu$M), the addition of Glimepiride at doses of 100-500 $\mu$M increased the anticancer activity of Doxorubicin, which was greater than with each agent alone or the sum of their activity. The increase in activity of the drug combinations relative to Doxorubicin at the appropriate dose is shown in FIG. **17,** which demonstrates that the dose combinations tested have 1.2 to 3.6-fold greater efficacy in killing A549 cells than Doxorubicin alone.

The strongest synergistic effect (CI = 0.011-0.117) and the highest increase in anticancer activity (194-363% compared to Doxorubicin alone) is observed when Doxorubicin is administrated at $IC_{50}$ (0.145 $\mu$M) and 2 times $IC_{50}$ (0.29 $\mu$M). For example, when 100 $\mu$M Glimepiride is administered together with 0.29 $\mu$M Doxorubicin, there is a reduction in cancer cell survival from 48.5% to 26.5%, corresponding to a 1.8-fold increase in anticancer activity. Another example shows that when 100 $\mu$M Glimepiride is administered in combination with 0.58 $\mu$M Doxorubicin, there is an even greater reduction in cancer cell survival from 27.0% to 9.4%, corresponding to a 2.9-fold increase in anticancer activity against A549 cells compared to Doxorubicin alone. In contrast, FIGS. **18-20** show that when doses of Doxorubicin (0.073, 0.145, and 0.290 $\mu$M) and Glimepiride (95.1, 190.3, 380.5 $\mu$M) were reduced to ¼ $IC_{50}$, ½ $IC_{50}$, and $IC_{50}$, respectively, the synergistic effect was weaker than at higher drug doses, but was still clearly noticeable and led to an increase in anticancer activity. Thus, there is a moderate to strong synergistic effect between glimepiride and doxorubicin on lung cancer cells, but the magnitude of this effect depends on the selection of appropriate doses of both drugs.

*Example 3*

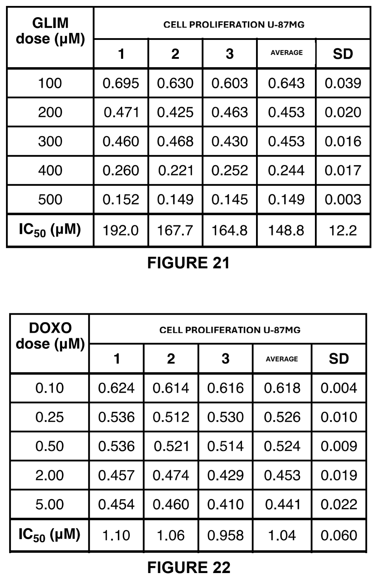

The same studies from Example **1** were repeated, but MCF-7 cells were replaced with U-87MG cells, a cell line that was isolated from malignant gliomas from a patient presumed to have glioblastoma. The results are shown in FIGS. **21-28.** As shown in FIG. **21,** increasing doses of Glimepiride alone resulted in decreased cell survival with an $IC_{50}$ value of 148.8$\pm$12.2 $\mu$M. FIG. **22** shows that Doxorubicin alone has an $IC_{50}$ value of 1.04$\pm$0.06 $\mu$M.

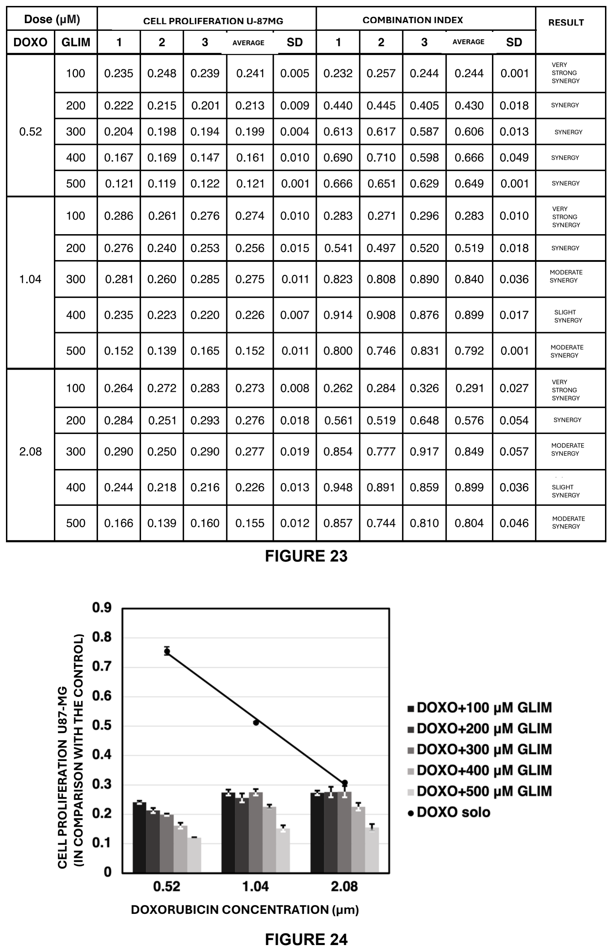

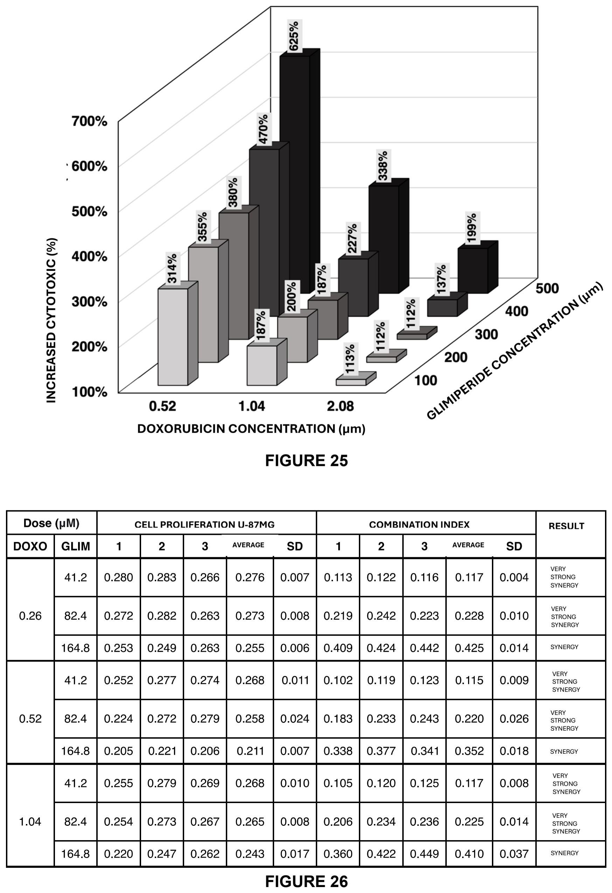

FIG. **23** shows that for all dose combinations of Doxorubicin and Glimepiride, CI<1 and ranges from 0.244$\pm$0.001 to 0.899$\pm$0.036, indicating a very strong synergistic effect between the two drugs against brain cancer cells. FIG. **24** shows that the antiproliferative activity of all tested combinations of Doxorubicin and Glimepiride against the U-87MG line far exceeds both the activity of the individual drugs and the sum of their activities. In studies with high doses of Doxorubicin (0.52-2.08 $\mu$M) and Glimepiride (100-500 $\mu$M), a similar response of U-87MG cells is observed regardless of the dose combination tested, that is, the fraction of viable cells dropped to a plateau of 0.155-0.277. Therefore, the greatest increase in antitumor activity for the two-drug combination relative to Doxorubicin alone is observed for the series with the lowest dose of Doxorubicin (0.52 $\mu$M). FIG. **25** show a clear trend toward an increase in the ratio of anticancer activity of the drug combination compared to Doxorubicin alone as the dose of Doxorubicin decreases. The combination of the lowest dose of Doxorubicin (0.52 $\mu$M) and Glimepiride (100 $\mu$M) resulted in a decrease in U-87MG cell survival from 75.6% for Doxorubicin alone to 24.1% for the combination of both drugs, corresponding to a 3.1-fold increase in the antitumor activity of the combination therapy compared to Doxorubicin alone.

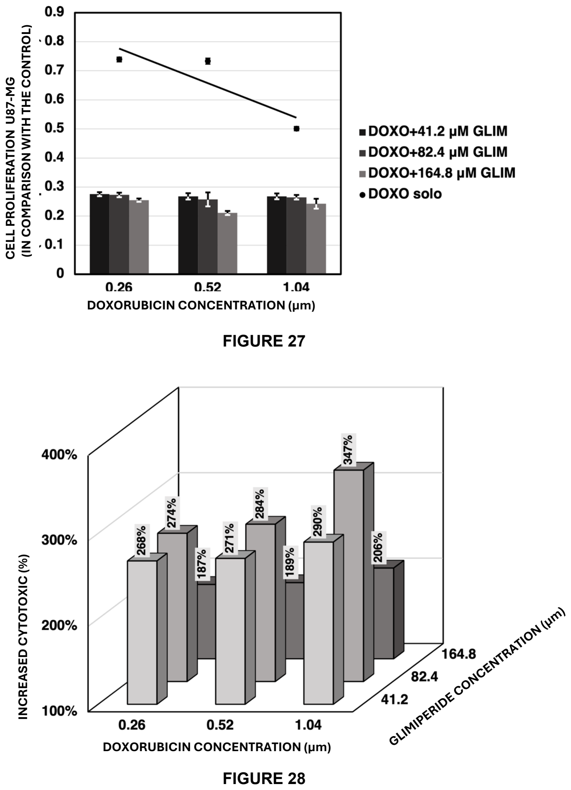

FIG. **26** shows a table with dose-response results for combinations of Doxorubicin (0.26, 0.52 and 1.04 $\mu$M) and Glimepiride (41.2, 82.4 and 164.8 $\mu$M) at low doses corresponding to their ¼ $IC_{50}$, ½ $IC_{50}$ and $IC_{50}$ against U-87MG cells. Lowering the doses of both drugs preserved the strong synergistic effect (CI = 0.115-0.425) and the enhancement of anticancer activity across the range of dose combinations tested. FIG. **27** shows that administration of each dose combination of Doxorubicin (0.26, 0.52 and 1.04 $\mu$M) and Glimepiride (41.2, 82.4 and 164.8 $\mu$M) results in inhibition of U-87MG cell proliferation at a similar level, i.e., about 25% compared to untreated control. Reaching a plateau at this level means that the most effective increase in anticancer activity is observed for the combination of the lowest dose of Doxorubicin (0.26 $\mu$M) and the lowest dose of Glimepiride (41.2 $\mu$M) tested. FIG. **28** shows that an approximately 2.7-fold increase in anticancer activity is observed for this combination compared to the antiproliferative effect induced by the corresponding dose of Doxorubicin alone (0.26 $\mu$M). These results indicate a very strong synergy between Doxorubicin and Glimepiride, resulting in increased antitumor activity of the combination therapy, which may find application in the treatment of patients with cancer, including brain tumors.

*Example 4*

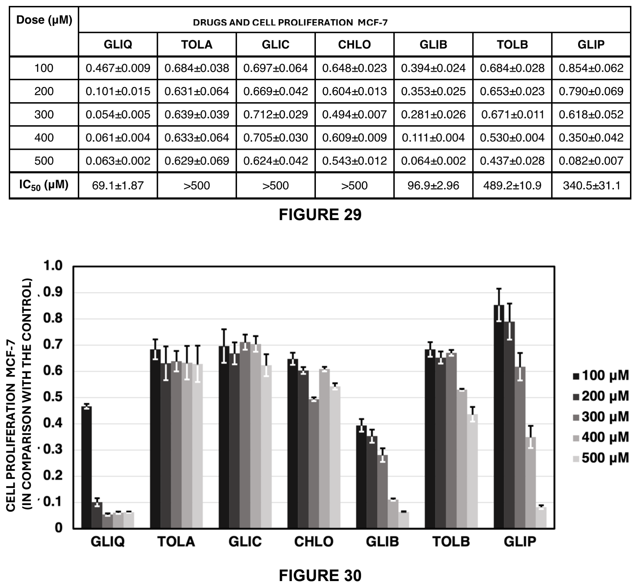

Considering the structural similarity between Glimepiride and its analogs used in diabetes therapy, in particular: Gliquidone, Glyburide, Glipizide, Gliclazide, Tolazamide, Chlorpropamide, Tolbutamide and Carbutamide, it is plausible that they could also find application in combination therapy with Doxorubicin, as could Glimepiride. For this reason, the above-mentioned Glimepiride analogs were tested in combination with various doses of Doxorubicin against the MCF-7 breast cancer cell line. The study was conducted similarly to Example **1.** The results are shown in FIGS. **29-33.**

FIG. **29** shows the dose/response table for Glimepiride analogs alone. Results are presented as mean$\pm$standard error for each Glimepiride dose tested. When possible, i.e., when $IC_{50}$<500 $\mu$M, the theoretical mean$\pm$standard error of $IC_{50}$ was calculated. FIG. **30** is a graphical representation of the results obtained. Most of the tested Glimepiride analogs, with the exception of Gliquidone and Glyburide, did not show significant cytotoxicity against MCF-7 cells in the tested dose range (100-500 $\mu$M). Thus, it can be assumed that the increased cytotoxicity of combination therapy involving Glimepiride analogs and Doxorubicin is not due to the combined cytotoxicity but to the synergistic effects of the two drugs administered.

Glimepiride analogs were tested against MCF-7 cells in combination with three various doses of Doxorubicin (0.225, 0.450 and 0.900 $\mu$M) corresponding to ¼ $IC_{50}$, ½ $IC_{50}$ and $IC_{50}$ of Doxorubicin against MCF-7 cells. FIG. **31** shows the results of combining Glimepiride analogs with Doxorubicin against MCF-7 cells. It can be clearly seen that most combinations of all the Glimepiride analogs tested show synergistic effects with Doxorubicin to varying degrees, suggesting that these derivatives may find application in anticancer therapy.

The assumption that the other Glimepiride analogs may be useful in combination therapy with Doxorubicin is not confirmed as Gliquidone, only show synergy with Doxorubicin against MCF-7 cells in certain dose combinations tested. FIG. **31** shows that only 100 $\mu$M Gliquidone in combination with Doxorubicin, shows additive effect or slight antagonism, but in other dose combinations Gliquidone shows synergistic effects with Doxorubicin. For this reason, the usefulness of any GLI analog in combination therapy with Doxorubicin against breast cancer or other types of cancer cannot be considered obvious. In contrast, all other Glimepiride analogs show a satisfactory level of synergy in combination with Doxorubicin. Of particular interest are analogs such as Chlorpropamide, Tolazamide, Gliclazide and Glyburide, which show strong or very strong synergy with Doxorubicin over a wide range of dose combinations. Such results suggest that these Glimepiride analogs may be particularly useful in combination therapy with Doxorubicin.

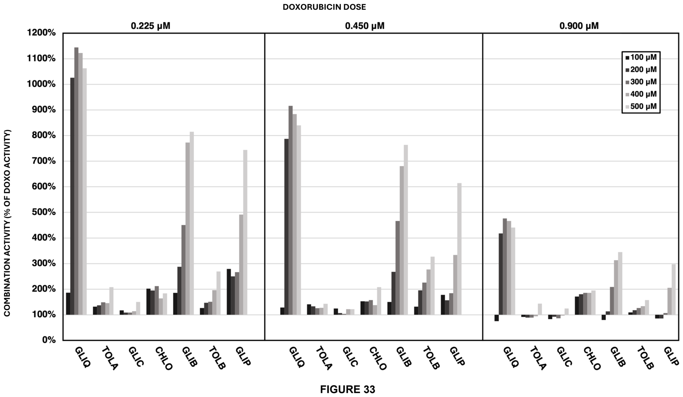

FIG. **32** shows that while both the Glimepiride analogs and Doxorubicin alone reduced the number of cells, in combination their effects were enhanced, indicating a synergistic effect. The dashed line in FIG. **32** indicates the fraction of viable cells remaining after treatment with the corresponding dose of Doxorubicin alone, therefore anything below this line indicates a better anticancer effect than with treatment with Doxorubicin alone. At the lowest dose of Doxorubicin tested (0.225 $\mu$M), all tested dose combinations of Glimepiride analogs significantly improve the anticancer effect of Doxorubicin. FIG. **33** shows that compared to the antiproliferative effect on MCF-7 cells induced by Doxorubicin alone, combination therapy results in up to an 11-fold increase in the antiproliferative effect. The study shows that the use of Glimepiride analogs in combination with Doxorubicin, specifically: Gliquidone, Glyburide, Glipizide, Gliclazide, Tolazamide, Chlorpropamide, Tolbutamide and Carbutamide, has a beneficial effect on increasing the anticancer activity of Doxorubicin. One possible application of such therapy is to improve the outcome of cancer patients treated with Doxorubicin. The invention could also be used to reduce the effective dose of Doxorubicin needed to produce the desired medical effect in cancer patients.

*Example 5*

The half maximal inhibitory concentration ($IC_{50}$) of GLIM for MCF-7, A549, and U-87MG cell lines were found to be 186.2 $\mu$M (FIG. **5),** 388.7 $\mu$M (FIG. **13),** and 148.8 $\mu$M (FIG. **21),** respectively. The $IC_{50}$ of DOCE for MCF-7, A549, and U-87MG cell lines were found to be 0.24 $\mu$M,0.15 $\mu$M, and 0.08 $\mu$M, respectively. In this study, we investigated the combined effects of GLIM and DOCE using various concentrations of each drug (i.e., ¼ $IC_{50}$, ½ $IC_{50}$, and $IC_{50}$) in MCF-7, A549, and U-87MG cell cultures. Specifically, we tested the following combinations: ¼ $IC_{50}$ GLIM + ¼ $IC_{50}$ DOCE, ¼ $IC_{50}$ GLIM + ½ $IC_{50}$ DOCE, ¼ $IC_{50}$ GLIM + $IC_{50}$ DOCE, ½ $IC_{50}$ GLIM + ¼ $IC_{50}$ DOCE, ½ $IC_{50}$ GLIM + ½ $IC_{50}$ DOCE, ½ $IC_{50}$ GLIM + $IC_{50}$ DOCE, $IC_{50}$ GLIM + ¼ $IC_{50}$ DOCE, $IC_{50}$ GLIM + ½ $IC_{50}$ DOCE, and $IC_{50}$ GLIM + $IC_{50}$ DOCE. The results of our experiments demonstrate that all tested combinations of GLIM and DOCE exhibit synergistic effect in inhibiting the proliferation of MCF-7, A549, and U-87MG cells. Therefore, the combination of GLIM and DOCE at various ratios as described herein may represent a promising therapeutic approach for treating cancer, particularly in breast cancer (MCF-7), lung cancer (A549), and glioblastoma (U-87MG).

*Example 6*

The half maximal inhibitory concentration ($IC_{50}$) of GLIM for MCF-7, A549, and U-87MG cell lines were found to be 186.2 $\mu$M (FIG. **5),** 388.7 $\mu$M (FIG. **13),** and 148.8 $\mu$M (FIG. **21),** respectively. The $IC_{50}$ of CAPE for MCF-7, A549, and U-87MG cell lines were found to be 0.18 $\mu$M, 0.06 $\mu$M, and 0.04 $\mu$M, respectively. In this study, we investigated the combined effects of GLIM and CAPE using various concentrations of each drug (i.e., ¼ $IC_{50}$, ½ $IC_{50}$, and $IC_{50}$) in MCF-7, A549, and U-87MG cell cultures. Specifically, we tested the following combinations: ¼ $IC_{50}$ GLIM + ¼ $IC_{50}$ CAPE, ¼ $IC_{50}$ GLIM + ½ $IC_{50}$ CAPE, ¼ $IC_{50}$ GLIM + $IC_{50}$ CAPE, ½ $IC_{50}$ GLIM + ¼ $IC_{50}$ CAPE, ½ $IC_{50}$ GLIM + ½ $IC_{50}$ CAPE, ½ $IC_{50}$ GLIM + $IC_{50}$ CAPE, $IC_{50}$ GLIM + ¼ $IC_{50}$ CAPE, $IC_{50}$ GLIM + ½ $IC_{50}$ CAPE, and $IC_{50}$ GLIM + $IC_{50}$ CAPE. The results of our experiments demonstrate that all tested combinations of GLIM and CAPE exhibit synergistic effect in inhibiting the proliferation of MCF-7, A549, and U-87MG cells.

**Claims**

1.   A synergistic composition comprising a combination of

- Glimepiride or
- a Glimepiride analog selected from the group consisting of Tolazamide, Gliclazide, Chlorpropamide, Tolbutamide and Glipizide, and
- an anticancer agent selected from the group consisting of: Doxorubicin, Docetaxel and Capecitabine.

for use in the treatment of cancer

2.   The synergistic composition according to the preceding claim, comprising Doxorubicin and Glimepiride.

3. The synergistic composition according to the preceding claim comprising one or more of the following Glimepiride analogs Tolazamide, Gliclazide, Chlorpropamide, Tolbutamide and Glipizide.

4. The synergistic composition according to any of the preceding claims, wherein Glimepiride, and/or the Glimepiride analog is administered to a subject, and then, Doxorubicin is administered.

5. The synergistic composition according to any of the preceding claims 1 to 3, wherein Doxorubicin and Glimepiride, and/or the Glimepiride analog are administered simultaneously.

6. The synergistic composition according to any of the preceding claims 1 to 3, wherein Doxorubicin is administered first and Glimepiride, and/or the Glimepiride analog are administered after Doxorubicin.

7. The synergistic composition according to any of the preceding claims, wherein the cancer is selected from breast, lung, brain cancer, lymphoblastic leukemia, acute myeloblastic leukemia, Hodgkin lymphoma, non-Hodgkin lymphoma (NHL), metastatic breast cancer, metastatic Wilms' tumor, metastatic neuroblastoma, metastatic soft tissue sarcoma, metastatic bone sarcoma, metastatic ovarian carcinoma, metastatic transitional cell, bladder carcinoma, metastatic thyroid carcinoma, metastatic gastric carcinoma, metastatic bronchogenic carcinoma or combinations thereof.

8. The synergistic composition according to any of the preceding claims, wherein Doxorubicin concentration is in the range of 0.001 $\mu$g/ml to 10 $\mu$g/ml for use in the treatment of cancer

9. The synergistic composition according to any of the preceding claims 1 to 7, wherein Glimepiride concentration is in the range of 1 $\mu$g/ml to 1000 $\mu$g/ml for use in the treatment of cancer.

10. The synergistic composition according to any of the preceding claims, comprising 0.006 $\mu$g/ml to 2.75 $\mu$g/ml of Doxorubicin and 9.81 $\mu$g/ml to 490.64 $\mu$g/ml of Glimepiride for use in the treatment of cancer.

11. The synergistic composition according to any of the preceding claims 1 to 10, comprising 0.044 $\mu$g/ml to 0.083 $\mu$g/ml of Doxorubicin and 9.81 $\mu$g/ml to 49.1 $\mu$g/ml of Glimepiride for use in the treatment of cancer.

12. The synergistic composition according to any of the preceding claims 10 to 11, wherein the cancer is breast cancer.

13. The synergistic composition according to any of the preceding claims 1 to 9, comprising 0.006 $\mu$g/ml to 0.55 $\mu$g/ml of Doxorubicin and 9.81 $\mu$g/ml to 490.6 $\mu$g/ml of Glimepiride for use in the treatment of cancer.

14. The synergistic composition according to any of the preceding claims 1 to 9 and 13, comprising 0.17 $\mu$g/ml to 0.5 $\mu$g/ml of Doxorubicin and 24.53 $\mu$g/ml to 85.86 $\mu$g/ml of Glimepiride for use in the treatment of cancer.

15. The synergistic composition according to any of the preceding claims 13 to 14, wherein the cancer is lung cancer.

16. The synergistic composition according to any of the preceding claims 1 to 9, comprising 0.055 $\mu$g/ml to 2.75 $\mu$g/ml of Doxorubicin and 9.812 $\mu$g/ml to 490.6 $\mu$g/ml of Glimepiride for use in the treatment of cancer.

17. The synergistic composition according to any of the preceding claims 1 to 9 and 16, comprising 0.11 $\mu$g/ml to 0.55 $\mu$g/ml of Doxorubicin and 19.62 $\mu$g/ml to 88.29 $\mu$g/ml of Glimepiride for use in the treatment of cancer.

18. The synergistic composition according to any of the preceding claims 16 to 17, wherein the cancer is brain cancer.

19. The synergistic composition according to any of the preceding claims, wherein Tolazamide concentration is in the range of 10 $\mu$g/ml to 300 $\mu$g/ml.

20. The synergistic composition according to any of the preceding claims, wherein Gliclazide concentration is in the range of 10 $\mu$g/ml to 300 $\mu$g/ml.

21. The synergistic composition according to any of the preceding claims, wherein Chlorpropamide concentration is in the range of 10 $\mu$g/ml to 300 $\mu$g/ml.

22. The synergistic composition according to any of the preceding claims, wherein Tolbutamide concentration is in the range of 10 $\mu$g/ml to 300 $\mu$g/ml.

23. The synergistic composition according to any of the preceding claims, wherein Glipizide concentration is in the range of 10 $\mu$g/ml to 300 $\mu$g/ml.

24. The synergistic composition according to any of the preceding claims, wherein Doxorubicin and Glimepiride, and/or the Glimepiride analog are in the form of an aqueous dispersion, gel, ampule, powder, capsule, pill, tablet, or combination thereof.

25. The synergistic composition according to any of the preceding claims, wherein the subject is a human cancer patient.

26. The synergistic composition according to any of the preceding claims, wherein the subject is being administered chemotherapy and/or radiotherapy before, simultaneously or subsequently to the administration of Doxorubicin and Glimepiride, and/or the Glimepiride analog.

27. The synergistic composition according to any of the preceding claims, wherein the combination of Doxorubicin and Glimepiride and/or a Glimepiride analog comprise between 20% - 99.5% by weight.

28. The synergistic composition according to claim 1, comprising Docetaxel and Glimepiride.

29. The synergistic composition according to the preceding claim, comprising 0.081 $\mu$g/ml to 0.405 $\mu$g/ml of Docetaxel and 9.81 $\mu$g/ml to 245.30 $\mu$g/ml of Glimepiride for use in the treatment of cancer.

30. The synergistic composition according to claim 1, comprising Capecitabine and Glimepiride.

31. The synergistic composition according to the preceding claim, comprising 0.0036 $\mu$g/ml to 0.18 $\mu$g/ml of Capecitabine and 9.81 $\mu$g/ml to 245.30 $\mu$g/ml of Glimepiride for use in the treatment of cancer.

32. A pharmaceutical composition comprising the synergistic combination disclosed in any of claims 1 to 31, and

    - one or more additional active compound, and/or
    - one or more pharmaceutically acceptable carrier, adjuvant, or vehicle

    for use in the treatment of cancer.

33. The pharmaceutical composition according to the preceding claim, wherein the one or more additional active compound is selected from doxorubicin, capecitabine, docetaxel, cytosine arabinoside, vincristine, prednisone, L-asparaginase, arabinosyl cytosine, 5-fluorouracil, cyclophosphamide, methotrexate, procarbazine, cisplatinum, etoposide, bleomycin, dacarbazine, and mitomycin-C or combinations thereof.

34. The pharmaceutical composition according to any of the preceding claims 32 to 33, that is administered vaginally, rectally, intranasally, orally, by inhalation, or parenterally.

35. The pharmaceutical composition according to the preceding claim, wherein the parenteral administration is intravenously.

**FIGURE 1**

**FIGURE 2**

**FIGURE 3**

| COMPOUD STRUCTURE | GENERIC NAME | TRADEMARK |
|---|---|---|
| | Glimepiride | Amaryl |
| | Gliquidone | Glurenorm |
| | Glyburide | Glynase |
| | Glipizide | Glucotrol |
| | Gliclazide | Diamicron |
| | Tolazamide | Tolinase |
| | Chlorpropamide | Diabinese |
| | Tolbutaminde | Tolbutamide |
| | Carbutamide | Glucidoral |

**FIGURE 4**

| GLIM dose (μM) | CELL PROLIFERATION MCF-7 | | | | |
| --- | --- | --- | --- | --- | --- |
| | **1** | **2** | **3** | AVERAGE | **SD** |
| 100 | 0.600 | 0.591 | 0.573 | 0.588 | 0.011 |
| 200 | 0.494 | 0.477 | 0.485 | 0.485 | 0.004 |
| 300 | 0.474 | 0.459 | 0.456 | 0.463 | 0.008 |
| 400 | 0.387 | 0.409 | 0.397 | 0.398 | 0.009 |
| 500 | 0.298 | 0.293 | 0.296 | 0.296 | 0.002 |
| $IC_{50}$ (μM) | 194.9 | 185.9 | 177.7 | 186.2 | 7.02 |

**FIGURE 5**

| DOXO dose (μM) | CELL PROLIFERATION MCF-7 | | | | |
| --- | --- | --- | --- | --- | --- |
| | **1** | **2** | **3** | AVERAGE | **SD** |
| 0.10 | 0.793 | 0.893 | 0.837 | 0.841 | 0.041 |
| 0.25 | 0.736 | 0.756 | 0.718 | 0.737 | 0.016 |
| 0.50 | 0.449 | 0.414 | 0.389 | 0.417 | 0.025 |
| 2.00 | 0.397 | 0.385 | 0.387 | 0.390 | 0.005 |
| 5.00 | 0.347 | 0.312 | 0.299 | 0.319 | 0.020 |
| $IC_{50}$ (μM) | 0.455 | 0.441 | 0.467 | 0.454 | 0.011 |

**FIGURE 6**

| Dose (µM) | | CELL PROLIFERATION MCF-7 | | | | | COMBINATION INDEX | | | | | RESULT |
|---|---|---|---|---|---|---|---|---|---|---|---|---|
| DOXO | GLIM | 1 | 2 | 3 | AVERAGE | SD | 1 | 2 | 3 | AVERAGE | SD | |
| 0.225 | 100 | 0.117 | 0.117 | 0.157 | 0.130 | 0.019 | 0.035 | 0.039 | 0.058 | 0.044 | 0.010 | VERY STRONG SYNERGY |
| | 200 | 0.128 | 0.151 | 0.174 | 0.151 | 0.019 | 0.075 | 0.102 | 0.121 | 0.099 | 0.019 | VERY STRONG SYNERGY |
| | 300 | 0.108 | 0.140 | 0.167 | 0.138 | 0.024 | 0.083 | 0.124 | 0.157 | 0.122 | 0.030 | STRONG SYNERGY |
| | 400 | 0.107 | 0.097 | 0.152 | 0.119 | 0.024 | 0.109 | 0.087 | 0.170 | 0.122 | 0.035 | STRONG SYNERGY |
| | 500 | 0.089 | 0.074 | 0.130 | 0.098 | 0.024 | 0.101 | 0.069 | 0.157 | 0.110 | 0.037 | VERY STRONG SYNERGY |
| 0.450 | 100 | 0.089 | 0.036 | 0.165 | 0.097 | 0.053 | 0.025 | 0.008 | 0.083 | 0.039 | 0.032 | VERY STRONG SYNERGY |
| | 200 | 0.116 | 0.135 | 0.127 | 0.126 | 0.08 | 0.069 | 0.100 | 0.079 | 0.083 | 0.013 | VERY STRONG SYNERGY |
| | 300 | 0.132 | 0.102 | 0.151 | 0.128 | 0.020 | 0.123 | 0.084 | 0.146 | 0.118 | 0.025 | STRONG SYNERGY |
| | 400 | 0.116 | 0.078 | 0.146 | 0.113 | 0.028 | 0.129 | 0.067 | 0.173 | 0.123 | 0.043 | STRONG SYNERGY |
| | 500 | 0.077 | 0.109 | 0.131 | 0.105 | 0.022 | 0.082 | 0.139 | 0.171 | 0.131 | 0.037 | STRONG SYNERGY |
| 0.900 | 100 | 0.125 | 0.140 | 0.174 | 0.146 | 0.020 | 0.056 | 0.105 | 0.133 | 0.098 | 0.032 | VERY STRONG SYNERGY |
| | 200 | 0.120 | 0.114 | 0.144 | 0.126 | 0.013 | 0.002 | 0.100 | 0.128 | 0.077 | 0.054 | VERY STRONG SYNERGY |
| | 300 | 0.130 | 0.094 | 0.149 | 0.124 | 0.023 | 0.132 | 0.092 | 0.173 | 0.132 | 0.033 | STRONG SYNERGY |
| | 400 | 0.106 | 0.093 | 0.137 | 0.112 | 0.019 | 0.119 | 0.108 | 0.182 | 0.137 | 0.032 | STRONG SYNERGY |
| | 500 | 0.099 | 0.093 | 0.121 | 0.105 | 0.012 | 0.130 | 0.127 | 0.170 | 0.142 | 0.020 | STRONG SYNERGY |

**FIGURE 7**

**FIGURE 8**

**FIGURE 9**

| Dose (µM) | | CELL PROLIFERATION MCF-7 | | | | | COMBINATION INDEX | | | | | RESULT |
|---|---|---|---|---|---|---|---|---|---|---|---|---|
| DOXO | GLIM | 1 | 2 | 3 | AVERAGE | SD | 1 | 2 | 3 | AVERAGE | SD | |
| 0.113 | 46.6 | 0.152 | 0.162 | 0.167 | 0.160 | 0.006 | 0.026 | 0.032 | 0.031 | 0.030 | 0.002 | VERY STRONG SYNERGY |
| | 93.1 | 0.133 | 0.160 | 0.138 | 0.144 | 0.012 | 0.038 | 0.053 | 0.037 | 0.043 | 0.007 | VERY STRONG SYNERGY |
| | 186.2 | 0.141 | 0.158 | 0.143 | 0.147 | 0.008 | 0.080 | 0.093 | 0.072 | 0.082 | 0.009 | VERY STRONG SYNERGY |
| 0.225 | 46.6 | 0.149 | 0.159 | 0.140 | 0.149 | 0.008 | 0.029 | 0.042 | 0.029 | 0.033 | 0.006 | VERY STRONG SYNERGY |
| | 93.1 | 0.141 | 0.166 | 0.145 | 0.151 | 0.011 | 0.025 | 0.068 | 0.047 | 0.047 | 0.018 | VERY STRONG SYNERGY |
| | 186.2 | 0.150 | 0.145 | 0.140 | 0.145 | 0.004 | 0.092 | 0.090 | 0.076 | 0.086 | 0.007 | VERY STRONG SYNERGY |
| 0.450 | 46.6 | 0.141 | 0.149 | 0.138 | 0.143 | 0.005 | 0.034 | 0.057 | 0.042 | 0.044 | 0.010 | VERY STRONG SYNERGY |
| | 93.1 | 0.140 | 0.157 | 0.144 | 0.147 | 0.007 | 0.052 | 0.083 | 0.061 | 0.065 | 0.001 | VERY STRONG SYNERGY |
| | 186.2 | 0.126 | 0.155 | 0.136 | 0.139 | 0.012 | 0.075 | 0.121 | 0.085 | 0.094 | 0.020 | VERY STRONG SYNERGY |

**FIGURE 10**

**FIGURE 11**

FIGURE 12

| GLIM dose (µM) | CELL PROLIFERATION A549 | | | | |
|---|---|---|---|---|---|
| | 1 | 2 | 3 | AVERAGE | SD |
| 100 | 0.678 | 0.658 | 0.707 | 0.681 | 0.020 |
| 200 | 0.629 | 0.615 | 0.598 | 0.614 | 0.013 |
| 300 | 0.613 | 0.609 | 0.580 | 0.601 | 0.015 |
| 400 | 0.500 | 0.513 | 0.500 | 0.504 | 0.006 |
| 500 | 0.391 | 0.403 | 0.394 | 0.396 | 0.005 |
| $IC_{50}$ (µM) | 383.2 | 399.7 | 383.2 | 388.7 | 4.49 |

FIGURE 13

| DOXO dose (µM) | CELL PROLIFERATION A549 | | | | |
|---|---|---|---|---|---|
| | 1 | 2 | 3 | AVERAGE | SD |
| 0.10 | 0.591 | 0.576 | 0.568 | 0.578 | 0.010 |
| 0.25 | 0.514 | 0.500 | 0.508 | 0.507 | 0.006 |
| 0.50 | 0.471 | 0.459 | 0.432 | 0.454 | 0.016 |
| 2.00 | 0.418 | 0.371 | 0.350 | 0.380 | 0.028 |
| 5.00 | 0.289 | 0.250 | 0.255 | 0.265 | 0.017 |
| $IC_{50}$ (µM) | 0.350 | 0.274 | 0.246 | 0.290 | 0.044 |

FIGURA 14

| Dose (µM) | | CELL PROLIFERATION A549 | | | | | COMBINATION INDEX | | | | | RESULT |
|---|---|---|---|---|---|---|---|---|---|---|---|---|
| DOXO | GLIM | 1 | 2 | 3 | AVERAGE | SD | 1 | 2 | 3 | AVERAGE | SD | |
| 0.145 | 100 | 0.497 | 0.539 | 0.554 | 0.530 | 0.024 | 0.643 | 1.006 | 1.037 | 0.895 | 0.179 | SLIGHT SYNERGY |
| | 200 | 0.446 | 0.498 | 0.526 | 0.490 | 0.033 | 0.537 | 1.030 | 1.108 | 0.892 | 0.253 | SLIGHT SYNERGY |
| | 300 | 0.411 | 0.401 | 0.425 | 0.412 | 0.010 | 0.522 | 0.590 | 0.795 | 0.636 | 0.116 | SYNERGY |
| | 400 | 0.366 | 0.358 | 0.320 | 0.315 | 0.038 | 0.445 | 0.528 | 0.462 | 0.478 | 0.036 | SYNERGY |
| | 500 | 0.269 | 0.242 | 0.274 | 0.262 | 0.014 | 0.231 | 0.254 | 0.402 | 0.296 | 0.076 | VERY STRONG SYNERGY |
| 0.290 | 100 | 0.238 | 0.269 | 0.287 | 0.265 | 0.020 | 0.048 | 0.113 | 0.158 | 0.106 | 0.045 | VERY STRONG SYNERGY |
| | 200 | 0.226 | 0.213 | 0.202 | 0.214 | 0.010 | 0.070 | 0.095 | 0.105 | 0.090 | 0.015 | SYNERGY |
| | 300 | 0.203 | 0.219 | 0.190 | 0.204 | 0.012 | 0.077 | 0.141 | 0.132 | 0.117 | 0.028 | VERY STRONG SYNERGY |
| | 400 | 0.134 | 0.157 | 0.173 | 0.155 | 0.016 | 0.040 | 0.091 | 0.143 | 0.091 | 0.042 | VERY STRONG SYNERGY |
| | 500 | 0.127 | 0.153 | 0.145 | 0.142 | 0.011 | 0.044 | 0.106 | 0.129 | 0.093 | 0.036 | VERY STRONG SYNERGY |
| 0.580 | 100 | 0.100 | 0.089 | 0.094 | 0.094 | 0.004 | 0.006 | 0.010 | 0.016 | 0.011 | 0.004 | VERY STRONG SYNERGY |
| | 200 | 0.076 | 0.083 | 0.144 | 0.101 | 0.031 | 0.007 | 0.016 | 0.061 | 0.028 | 0.024 | VERY STRONG SYNERGY |
| | 300 | 0.081 | 0.083 | 0.095 | 0.086 | 0.006 | 0.011 | 0.023 | 0.041 | 0.025 | 0.012 | VERY STRONG SYNERGY |
| | 400 | 0.085 | 0.101 | 0.128 | 0.105 | 0.018 | 0.016 | 0.042 | 0.089 | 0.049 | 0.030 | VERY STRONG SYNERGY |
| | 500 | 0.117 | 0.152 | 0.130 | 0.133 | 0.014 | 0.038 | 0.112 | 0.113 | 0.088 | 0.035 | VERY STRONG SYNERGY |

## FIGURE 15

## FIGURA 16

FIGURE 17

| Dose (µM) | | CELL PROLIFERATION A549 | | | | | COMBINATION INDEX | | | | | RESULT |
|---|---|---|---|---|---|---|---|---|---|---|---|---|
| DOXO | GLIM | 1 | 2 | 3 | AVERAGE | SD | 1 | 2 | 3 | AVERAGE | SD | |
| 0.073 | 95.1 | 0.557 | 0.634 | 0.556 | 0.582 | 0.036 | 0.880 | 0.916 | 0.879 | 0.892 | 0.017 | SLIGHT SYNERGY |
| | 190.3 | 0.470 | 0.472 | 0.478 | 0.473 | 0.003 | 0.524 | 0.612 | 0.699 | 0.612 | 0.071 | SYNERGY |
| | 380.5 | 0.423 | 0.421 | 0.439 | 0.428 | 0.008 | 0.626 | 0.716 | 0.904 | 0.749 | 0.116 | MODERATE SYNERGY |
| 0.145 | 95.1 | 0.529 | 0.532 | 0.538 | 0.533 | 0.004 | 0.895 | 0.903 | 0.900 | 0.899 | 0.145 | SLIGHT SYNERGY |
| | 190.3 | 0.442 | 0.439 | 0.439 | 0.440 | 0.001 | 0.501 | 0.602 | 0.666 | 0.590 | 0.068 | SYNERGY |
| | 380.5 | 0.361 | 0.362 | 0.363 | 0.362 | 0.001 | 0.409 | 0.528 | 0.603 | 0.513 | 0.080 | SYNERGY |
| 0.290 | 95.1 | 0.241 | 0.244 | 0.234 | 0.239 | 0.004 | 0.049 | 0.083 | 0.087 | 0.073 | 0.017 | VERY STRONG SYNERGY |
| | 190.3 | 0.226 | 0.219 | 0.228 | 0.224 | 0.004 | 0.067 | 0.098 | 0.132 | 0.099 | 0.027 | VERY STRONG SYNERGY |
| | 380.5 | 0.129 | 0.112 | 0.115 | 0.119 | 0.007 | 0.035 | 0.052 | 0.069 | 0.052 | 0.014 | VERY STRONG SYNERGY |

FIGURE 18

**FIGURE 19**

**FIGURE 20**

| GLIM dose (µM) | CELL PROLIFERATION U-87MG | | | | |
|---|---|---|---|---|---|
| | **1** | **2** | **3** | AVERAGE | **SD** |
| 100 | 0.695 | 0.630 | 0.603 | 0.643 | 0.039 |
| 200 | 0.471 | 0.425 | 0.463 | 0.453 | 0.020 |
| 300 | 0.460 | 0.468 | 0.430 | 0.453 | 0.016 |
| 400 | 0.260 | 0.221 | 0.252 | 0.244 | 0.017 |
| 500 | 0.152 | 0.149 | 0.145 | 0.149 | 0.003 |
| **$IC_{50}$ (µM)** | 192.0 | 167.7 | 164.8 | 148.8 | 12.2 |

**FIGURE 21**

| DOXO dose (µM) | CELL PROLIFERATION U-87MG | | | | |
|---|---|---|---|---|---|
| | **1** | **2** | **3** | AVERAGE | **SD** |
| 0.10 | 0.624 | 0.614 | 0.616 | 0.618 | 0.004 |
| 0.25 | 0.536 | 0.512 | 0.530 | 0.526 | 0.010 |
| 0.50 | 0.536 | 0.521 | 0.514 | 0.524 | 0.009 |
| 2.00 | 0.457 | 0.474 | 0.429 | 0.453 | 0.019 |
| 5.00 | 0.454 | 0.460 | 0.410 | 0.441 | 0.022 |
| **$IC_{50}$ (µM)** | 1.10 | 1.06 | 0.958 | 1.04 | 0.060 |

**FIGURE 22**

| Dose (µM) | | CELL PROLIFERATION U-87MG | | | | | COMBINATION INDEX | | | | | RESULT |
|---|---|---|---|---|---|---|---|---|---|---|---|---|
| DOXO | GLIM | 1 | 2 | 3 | AVERAGE | SD | 1 | 2 | 3 | AVERAGE | SD | |
| 0.52 | 100 | 0.235 | 0.248 | 0.239 | 0.241 | 0.005 | 0.232 | 0.257 | 0.244 | 0.244 | 0.001 | VERY STRONG SYNERGY |
| | 200 | 0.222 | 0.215 | 0.201 | 0.213 | 0.009 | 0.440 | 0.445 | 0.405 | 0.430 | 0.018 | SYNERGY |
| | 300 | 0.204 | 0.198 | 0.194 | 0.199 | 0.004 | 0.613 | 0.617 | 0.587 | 0.606 | 0.013 | SYNERGY |
| | 400 | 0.167 | 0.169 | 0.147 | 0.161 | 0.010 | 0.690 | 0.710 | 0.598 | 0.666 | 0.049 | SYNERGY |
| | 500 | 0.121 | 0.119 | 0.122 | 0.121 | 0.001 | 0.666 | 0.651 | 0.629 | 0.649 | 0.001 | SYNERGY |
| 1.04 | 100 | 0.286 | 0.261 | 0.276 | 0.274 | 0.010 | 0.283 | 0.271 | 0.296 | 0.283 | 0.010 | VERY STRONG SYNERGY |
| | 200 | 0.276 | 0.240 | 0.253 | 0.256 | 0.015 | 0.541 | 0.497 | 0.520 | 0.519 | 0.018 | SYNERGY |
| | 300 | 0.281 | 0.260 | 0.285 | 0.275 | 0.011 | 0.823 | 0.808 | 0.890 | 0.840 | 0.036 | MODERATE SYNERGY |
| | 400 | 0.235 | 0.223 | 0.220 | 0.226 | 0.007 | 0.914 | 0.908 | 0.876 | 0.899 | 0.017 | SLIGHT SYNERGY |
| | 500 | 0.152 | 0.139 | 0.165 | 0.152 | 0.011 | 0.800 | 0.746 | 0.831 | 0.792 | 0.001 | MODERATE SYNERGY |
| 2.08 | 100 | 0.264 | 0.272 | 0.283 | 0.273 | 0.008 | 0.262 | 0.284 | 0.326 | 0.291 | 0.027 | VERY STRONG SYNERGY |
| | 200 | 0.284 | 0.251 | 0.293 | 0.276 | 0.018 | 0.561 | 0.519 | 0.648 | 0.576 | 0.054 | SYNERGY |
| | 300 | 0.290 | 0.250 | 0.290 | 0.277 | 0.019 | 0.854 | 0.777 | 0.917 | 0.849 | 0.057 | MODERATE SYNERGY |
| | 400 | 0.244 | 0.218 | 0.216 | 0.226 | 0.013 | 0.948 | 0.891 | 0.859 | 0.899 | 0.036 | SLIGHT SYNERGY |
| | 500 | 0.166 | 0.139 | 0.160 | 0.155 | 0.012 | 0.857 | 0.744 | 0.810 | 0.804 | 0.046 | MODERATE SYNERGY |

**FIGURE 23**

**FIGURE 24**

EP 4 717 267 A1

FIGURE 25

| Dose (µM) | | CELL PROLIFERATION U-87MG | | | | | COMBINATION INDEX | | | | | RESULT |
|---|---|---|---|---|---|---|---|---|---|---|---|---|
| DOXO | GLIM | 1 | 2 | 3 | AVERAGE | SD | 1 | 2 | 3 | AVERAGE | SD | |
| 0.26 | 41.2 | 0.280 | 0.283 | 0.266 | 0.276 | 0.007 | 0.113 | 0.122 | 0.116 | 0.117 | 0.004 | VERY STRONG SYNERGY |
| | 82.4 | 0.272 | 0.282 | 0.263 | 0.273 | 0.008 | 0.219 | 0.242 | 0.223 | 0.228 | 0.010 | VERY STRONG SYNERGY |
| | 164.8 | 0.253 | 0.249 | 0.263 | 0.255 | 0.006 | 0.409 | 0.424 | 0.442 | 0.425 | 0.014 | SYNERGY |
| 0.52 | 41.2 | 0.252 | 0.277 | 0.274 | 0.268 | 0.011 | 0.102 | 0.119 | 0.123 | 0.115 | 0.009 | VERY STRONG SYNERGY |
| | 82.4 | 0.224 | 0.272 | 0.279 | 0.258 | 0.024 | 0.183 | 0.233 | 0.243 | 0.220 | 0.026 | VERY STRONG SYNERGY |
| | 164.8 | 0.205 | 0.221 | 0.206 | 0.211 | 0.007 | 0.338 | 0.377 | 0.341 | 0.352 | 0.018 | SYNERGY |
| 1.04 | 41.2 | 0.255 | 0.279 | 0.269 | 0.268 | 0.010 | 0.105 | 0.120 | 0.125 | 0.117 | 0.008 | VERY STRONG SYNERGY |
| | 82.4 | 0.254 | 0.273 | 0.267 | 0.265 | 0.008 | 0.206 | 0.234 | 0.236 | 0.225 | 0.014 | VERY STRONG SYNERGY |
| | 164.8 | 0.220 | 0.247 | 0.262 | 0.243 | 0.017 | 0.360 | 0.422 | 0.449 | 0.410 | 0.037 | SYNERGY |

FIGURE 26

**FIGURE 27**

**FIGURE 28**

| Dose (µM) | DRUGS AND CELL PROLIFERATION MCF-7 | | | | | | |
|---|---|---|---|---|---|---|---|
| | GLIQ | TOLA | GLIC | CHLO | GLIB | TOLB | GLIP |
| 100 | 0.467±0.009 | 0.684±0.038 | 0.697±0.064 | 0.648±0.023 | 0.394±0.024 | 0.684±0.028 | 0.854±0.062 |
| 200 | 0.101±0.015 | 0.631±0.064 | 0.669±0.042 | 0.604±0.013 | 0.353±0.025 | 0.653±0.023 | 0.790±0.069 |
| 300 | 0.054±0.005 | 0.639±0.039 | 0.712±0.029 | 0.494±0.007 | 0.281±0.026 | 0.671±0.011 | 0.618±0.052 |
| 400 | 0.061±0.004 | 0.633±0.064 | 0.705±0.030 | 0.609±0.009 | 0.111±0.004 | 0.530±0.004 | 0.350±0.042 |
| 500 | 0.063±0.002 | 0.629±0.069 | 0.624±0.042 | 0.543±0.012 | 0.064±0.002 | 0.437±0.028 | 0.082±0.007 |
| $IC_{50}$ (µM) | 69.1±1.87 | >500 | >500 | >500 | 96.9±2.96 | 489.2±10.9 | 340.5±31.1 |

**FIGURE 29**

**FIGURE 30**

| COMBINATION | | DRUG DOSE AND CELL PROLIFERATION MCF-7 | | | | | DRUG DOSE AND COMBINATION INDEX | | | | |
|---|---|---|---|---|---|---|---|---|---|---|---|
| DOXO | DRUG | 100 µM | 200 µM | 300 µM | 400 µM | 500 µM | 100 µM | 200 µM | 300 µM | 400 µM | 500 µM |
| | GLIQ | 0.319±0.022 | 0.058±0.001 | 0.052±0.001 | 0.053±0.001 | 0.056±0.001 | 0.957±0.033 | 0.515±0.018 | 0.717±0.025 | 0.971±0.023 | 1.261±0.039 |
| | TOLA | 0.452±0.034 | 0.433±0.036 | 0.399±0.035 | 0.409±0.050 | 0.286±0.022 | 0.185±0.028 | 0.164±0.022 | 0.131±0.014 | 0.146±0.032 | 0.054±0.003 |
| | GLIC | 0.508±0.043 | 0.548±0.056 | 0.547±0.033 | 0.520±0.054 | 0.396±0.029 | 0.259±0.075 | 0.378±0.164 | 0.368±0.108 | 0.344±0.176 | 0.131±0.045 |
| 0.225 | CHLO | 0.294±0.010 | 0.305±0.009 | 0.281±0.012 | 0.363±0.032 | 0.324±0.023 | 0.061±0.012 | 0.071±0.013 | 0.062±0.004 | 0.166±0.070 | 0.116±0.070 |
| | GLIB | 0.320±0.028 | 0.207±0.016 | 0.132±0.001 | 0.077±0.006 | 0.073±0.002 | 0.670±0.089 | 0.797±0.054 | 0.791±0.019 | 0.677±0.043 | 0.803±0.015 |
| | TOLB | 0.469±0.007 | 0.404±0.015 | 0.395±0.030 | 0.303±0.011 | 0.211±0.006 | 0.343±0.015 | 0.318±0.048 | 0.394±0.110 | 0.240±0.057 | 0.137±0.033 |
| | GLIP | 0.213±0.040 | 0.238±0.028 | 0.223±0.027 | 0.121±0.006 | 0.080±0.005 | 0.244±0.024 | 0.490±0.032 | 0.689±0.050 | 0.651±0.069 | 0.664±0.076 |
| | GLIQ | 0.393±0.008 | 0.064±0.002 | 0.055±0.001 | 0.057±0.001 | 0.060±0.002 | 1.319±0.069 | 0.551±0.006 | 0.749±0.016 | 1.016±0.014 | 1.316±0.019 |
| | TOLA | 0.357±0.030 | 0.379±0.026 | 0.400±0.026 | 0.396±0.033 | 0.353±0.035 | 0.183±0.018 | 0.217±0.014 | 0.254±0.017 | 0.251±0.027 | 0.183±0.019 |
| | GLIC | 0.404±0.029 | 0.472±0.015 | 0.486±0.036 | 0.414±0.022 | 0.414±0.022 | 0.253±0.069 | 0.398±0.057 | 0.451±0.125 | 0.276±0.065 | 0.280±0.069 |
| 0.450 | CHLO | 0.330±0.043 | 0.331±0.074 | 0.320±0.060 | 0.366±0.052 | 0.242±0.029 | 0.159±0.036 | 0.204±0.124 | 0.182±0.089 | 0.296±0.155 | 0.086±0.014 |
| | GLIB | 0.336±0.027 | 0.188±0.029 | 0.108±0.013 | 0.074±0.001 | 0.066±0.002 | 0.784±0.061 | 0.749±0.098 | 0.676±0.066 | 0.658±0.017 | 0.744±0.007 |
| | TOLB | 0.382±0.016 | 0.258±0.013 | 0.223±0.018 | 0.182±0.009 | 0.154±0.008 | 0.294±0.046 | 0.147±0.041 | 0.128±0.039 | 0.098±0.028 | 0.082±0.029 |
| | GLIP | 0.283±0.017 | 0.332±0.008 | 0.273±0.017 | 0.151±0.032 | 0.082±0.006 | 0.354±0.027 | 0.677±0.020 | 0.823±0.029 | 0.732±0.107 | 0.677±0.086 |
| | GLIQ | 0.317±0.019 | 0.057±0.003 | 0.050±0.001 | 0.051±0.001 | 0.054±0.001 | 1.153±0.071 | 0.519±0.028 | 0.707±0.019 | 0.947±0.021 | 1.231±0.034 |
| | TOLA | 0.260±0.029 | 0.267±0.023 | 0.268±0.029 | 0.252±0.019 | 0.166±0.007 | 0.172±0.008 | 0.183±0.025 | 0.189±0.053 | 0.161±0.018 | 0.070±0.019 |
| | GLIC | 0.286±0.017 | 0.257±0.018 | 0.275±0.007 | 0.245±0.018 | 0.191±0.004 | 0.211±0.040 | 0.172±0.057 | 0.196±0.047 | 0.156±0.052 | 0.091±0.022 |
| 0.900 | CHLO | 0.139±0.007 | 0.132±0.009 | 0.128±0.012 | 0.128±0.008 | 0.122±0.010 | 0.051±0.018 | 0.047±0.017 | 0.044±0.016 | 0.044±0.014 | 0.039±0.010 |
| | GLIB | 0.297±0.024 | 0.210±0.023 | 0.114±0.008 | 0.076±0.008 | 0.069±0.002 | 0.789±0.120 | 0.890±0.111 | 0.727±0.059 | 0.684±0.072 | 0.785±0.040 |
| | TOLB | 0.217±0.023 | 0.202±0.018 | 0.188±0.027 | 0.178±0.021 | 0.151±0.020 | 0.144±0.060 | 0.141±0.056 | 0.141±0.074 | 0.139±0.065 | 0.113±0.062 |
| | GLIP | 0.277±0.017 | 0.277±0.014 | 0.223±0.006 | 0.116±0.005 | 0.080±0.005 | 0.445±0.038 | 0.690±0.025 | 0.777±0.031 | 0.664±0.063 | 0.678±0.079 |

CI<0,10 Very strong synergy
0,10<CI<0,30 Strong synergy
0,30<CI<0,70 Synergy
0,70<CI<0,85 Moderate Synergy
0,85<CI<0,90 Slight Synergy
0,90<CI<1,10 Almost additive
1,10<CI<1,20 Slight antagonism
1,20<CI<1,45 Moderate antagonism
1,45<CI<3,30 Antagonism
3,30<CI<10,00 Strong Antagonism
CI>0,10 Very strong antagonism

FIGURE 31

DOXORUBICIN DOSE

FIGURE 32

FIGURE 33

# INTERNATIONAL SEARCH REPORT

| International application No. |
|---|
| PCT/ES2024/070255 |

## A. CLASSIFICATION OF SUBJECT MATTER

See extra sheet

According to International Patent Classification (IPC) or to both national classification and IPC

## B. FIELDS SEARCHED

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

EPODOC, INVENES, WPI, BIOSIS, EMBASE, MEDLINE, NPL, REGISTRY, CA

## C. DOCUMENTS CONSIDERED TO BE RELEVANT

| Category* | Citation of document, with indication, where appropriate, of the relevant passages | Relevant to claim No. |
|---|---|---|
| A | SUBRAMANIYAM NITHYANANTHAN et al.. Unveiling anticancer potential of glibenclamide: Its synergistic cytotoxicity with doxorubicin on cancer cells. Journal Of Pharmaceutical And Biomedical Analysis. May 30 2018., 30/05/2018, Vol. 154, páginas 294-301, ISSN 0731-7085(print), ISSN 1873-264X(electronic), <DOI: 10.1016/j.jpba.2018.03.025>; todo el documento, en especial conclusiones y resumen | 1 (en parte), 2 a 27, 32 a 35 (en parte) |
| A | NUÑEZ MARIEL et al.. Glibenclamide inhibits cell growth by inducing G0/G1 arrest in the human breast cancer cell line MDA-MB-231. Bmc Pharmacology & Toxicology, Jan 11 2013., 11/01/2013, Vol. 14, páginas Article No.: 6, ISSN 2050-6511(electronic), <DOI: 10.1186/2050-6511-14-6>; página 8, epígrafe "Combination treatments", página 11, figura 7C. | 1 (en parte), 2 a 27, 32 a 35 (en parte) |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
|---|---|
| 23/09/2024 | **(25/09/2024)** |

| Name and mailing address of the ISA/ | Authorized officer |
|---|---|
| OFICINA ESPAÑOLA DE PATENTES Y MARCAS<br>Paseo de la Castellana, 75 - 28071 Madrid (España)<br>Facsimile No.: 91 349 53 04 | E. Albarrán Gómez<br><br>Telephone No. 913493038 |

Form PCT/ISA/210 (second sheet) (July 2022)

## INTERNATIONAL SEARCH REPORT

International application No.

---

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

---

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

---

Form PCT/ISA/210 (continuation of first sheet (2)) (January 2015)

## INTERNATIONAL SEARCH REPORT

International application No.

PCT/ES2024/070255

| | C (continuation). | DOCUMENTS CONSIDERED TO BE RELEVANT | |
|---|---|---|---|
| Category * | Citation of documents, with indication, where appropriate, of the relevant passages | | Relevant to claim No. |
| A | UZMA FARIDI, Y COL.. An in-vitro and in Silico Anticancer Study of FDA Approved Antidiabetic Drugs Glimepiride and Empagliflozin. International Journal Of Lifescience And Pharma Research, 2020, Vol. 10, páginas 52-57, <DOI:10.22376/ijpbs/lpr.2020.10.2.L52-57> Todo el documento, en especial tabla 2 y figura 3. | | 1 (en parte), 2 a 27, 32 a 35 (en parte) |
| A | LONG LIANGYUAN et al.. The Anti-Breast Cancer Effect and Mechanism of Glimepiride-Metformin Adduct. Oncotargets And Therapy 2020., 30/11/2019, Vol. 13, páginas 3777-3788, ISSN 1178-6930(print) ISSN 1178-6930(electronic), <DOI: 10.2147/OTT.S240252> todo el documento, en especial página 3.782, figuras 2 y 3. | | 1 (en parte), 2 a 27, 32 a 35 (en parte) |
| A | LI YING et al.. Metformin synergistically suppress tumor growth with doxorubicin and reverse drug resistance by inhibiting the expression and function of P-glycoprotein in MCF7/ADR cells and xenograft models. Oncotarget Jan 5 2018., 05/01/2018, Vol. 9, N° 2, páginas 2158-2174, ISSN 1949-2553(electronic), <DOI: 10.18632/oncotarget.23187> página 2164, tabla 1; página 2166, figura 6, A y B, y también in vivo - página 2167, figura 7. | | 1 (en parte), 2 a 27, 32 a 35 (en parte) |
| A | GUIMARAES ISABELLA S et al.. Metformin inhibits proliferation and acts synergistically with paclitaxel and doxorubicin in triple negative breast cancer cell lines. Cancer Research Aug 1 2015., 31/07/2015, Vol. 75, N° Suppl. 15, páginas 2571, ISSN 0008-5472(print) ISSN 1538-7445(electronic), <DOI: 10.1158/1538-7445.AM2015-2571> todo el documento. | | 1 (en parte), 2 a 27, 32 a 35 (en parte) |
| A | ASENSIO-LOPEZ MARI C et al.. Metformin protects against doxorubicin-induced cardiotoxicity: Involvement of the adiponectin cardiac system. Free Radical Biology & Medicine Nov 15 2011, 15/11/2011, Vol. 51, N° 10, páginas 1861-1871, ISSN 0891-5849(print) ISSN 1873-4596(electronic), <DOI: 10.1016/j.freeradbiomed.2011.08.015> todo el documento. | | 1 (en parte), 2 a 27, 32 a 35 (en parte) |

Form PCT/ISA/210 (continuation of second sheet) (July 2022)

**INTERNATIONAL SEARCH REPORT**

International application No.

PCT/ES2024/070255

CLASSIFICATION OF SUBJECT MATTER

*A61K31/704* (2006.01)
*A61K31/64* (2006.01)
*A61P35/00* (2006.01)
*A61P35/04* (2006.01)

Form PCT/ISA/210 (extra sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **S. VOULGARIS et al.** *Am. J. Clin. Oncol.*, 2002, vol. 25, 60-64
- **M. NÚÑEZ et al.** *BMC Pharmacol. Toxicol.*, 2013, vol. 14, 6
- **ERTTMANN, R.** ; **ERB, N.** ; **STEINHOFF, A et al.** Pharmacokinetics of doxorubicin in man: dose and schedule dependence.. *J Cancer Res Clin Oncol*, 1988, vol. 114, 509-513
- **E. W. MARTIN**. Remington's Pharmaceutical Sciences. Mack Publishing Co, 1980
- **T.C. CHOU**. *Pharmacol. Rev*, 2006, vol. 58, 621-681
- **T.C. CHOU**. *J. Theor. Biol*, 1976, vol. 59, 253-276
- **T.C. CHOU**. *Integr. Biol.*, 2011, vol. 3, 548-559
- **T.C. CHOU** ; **P. TALALAY**. *Adv. Enz. Regul.*, 1984, vol. 22, 27-55